# EUROPEAN PATENT APPLICATION

(11) **EP 3 530 282 A1**
(43) Date of publication of application: **28.08.2019**
(21) Application number: 18305207.5
(22) Date of filing: 27.02.2018
(51) Int. Cl.: A61K 38/16, A61K 39/395, A61K 45/06, A61P 31/00, A61P 37/02

(54) **THERAPEUTIC METHODS**

(71) Applicant: Diaccurate, 75008 Paris (FR)
(72) Inventor: Pothlichet, Julien., 92310 Sèvres (FR); Pouletty, Phillippe., 75005 Paris (FR); Thèze, Jacques., 75007 Paris (FR)
(74) Representative: Cabinet Becker et Associés

(57) **Abstract**

The present invention relates to novel therapeutic approaches for treating or preventing diseases in mammals, particularly in human subjects, using a PLA2-GIB cofactor, or a modulator of a PLA2-GIB cofactor.

## Description

The present invention relates to novel therapeutic approaches for treating or preventing diseases in mammals, particularly in human subjects. The invention provides therapeutic methods based on the inhibition of a novel mechanism by which various pathogens act in mammals. The invention may be in used in a preventive or curative approach, alone or in combination with other treatments, against diseases caused by various pathogens such as viruses or bacteria.

### Introduction and background

It has been documented by the inventor that sPLA2-GIB is involved in the inactivation of CD4 T cells in HIV infected patients (see WO2015/097140). It was thus proposed and documented by the inventor that sPLA2-GIB modulators are effective for treating diseases in mammal, e.g., disorders associated with an immune deficiency.

Continuing their research, applicant has now found that the effect of sPLA2-GIB can be mediated and/or amplified by a cofactor present in diseased subjects, and that such cofactor acts through a gC1q receptor at the surface of T cells. In particular, the inventors have now shown that pathogens produce or activate a cofactor which binds to gClqR, leading to a sensitization of CD4 T cells to inactivation by very low doses of sPLA2-GIB. In patients infected with such pathogens, CD4 T cells become sensitive to inactivation by physiological amounts of sPLA2-GIB, while in non-infected subjects, CD4 T cells remain resistant to inactivation by such physiological concentration of sPLA2-GIB. The inventors have identified such gClqR-binding cofactors from various pathogens, including viruses or bacteria, such as HIV, HCV or S. aureus. Applicant also verified that said cofactors could sensitize CD4 T cells to inactivation by sPLA2-GIB, and that blocking of such cofactors in vivo could restore or maintain resistance of CD4 T cells to inactivation by sPLA2-GIB. Applicant thus identified a novel general mechanism by which many pathogens induce diseases or pathological conditions in mammals, i.e., by inducing a sensitization of CD4 T cells to inactivation by PLA2-GIB. Such unexpected findings allow applicant to provide novel therapeutic approaches based on a modulation of such cofactor, such as a blockade or inhibition thereof thereby preventing, avoiding or at least reducing the pathogenic effects of many pathogens.

### Summary of the invention

It is an object of the invention to provide methods for treating a mammalian subject, comprising administering to the subject a PLA2-GIB cofactor, or a modulator of a PLA2-GIB cofactor.

Another object of the invention relates to methods for modulating an immune response in a mammalian subject, comprising administering to the subject a PLA2-GIB cofactor, or a modulator of a PLA2-GIB co-factor.

Another object of the invention is a PLA2-GIB cofactor, or a modulator of a PLA2-GIB cofactor, for use for treating a mammalian subject.

The invention also relates to a PLA2-GIB cofactor, or a modulator of a PLA2-GIB co-factor, for use for modulating an immune response in a mammalian subject.

The PLA2-GIB cofactor may be a component of a pathogen or a nutrient, preferably a protein or peptide from or activated by a pathogen. In a particular embodiment, the PLA2-GIB cofactor is a viral or bacterial or fungal or parasite protein or peptide.

The modulator of PLA2-GIB cofactor may be an inhibitor of the PLA2-GIB cofactor, or an immunogen of the PLA2-GIB cofactor, or an activator, agonist or mimotope of the PLA2-GIB cofactor. Depending on the type of agent or modulator, the invention may be used e.g., to treat subjects having a condition requiring immunosuppressive or immunostimulating therapy.

The invention also relates to a combination therapy or therapeutic regimen for treating a disorder caused by a pathogen, comprising a combination of at least two active agents selected from (i) a drug active against the pathogen, (ii) a modulator of a PLA2-GIB cofactor, and (iii) a modulator of PLA2-GIB, said drug and modulator being for combined, separate or sequential administration.

The invention also relates to the use of a PLA2-GIB cofactor, or an agonist or mimotope thereof, for the manufacture of a medicament to induce immunosuppression in a subject in need thereof, by increasing the effect of PLA2-GIB on T cells.

The invention also relates to a modulator of a PLA2-GIB cofactor for use for modulating an immune response in a subject in need thereof, typically by modulating the effect of PLA2-GIB on T cells.

The invention also relates to the use of an inhibitor of a PLA2-GIB cofactor for the manufacture of a medicament to stimulate an immune response in a subject in need thereof.

In a further aspect, the invention relates to a method for selecting an immunostimulatory molecule, comprising testing whether a candidate molecule can inhibit gClqR-mediated sensitization of CD4 T cells.

The invention may be used in any mammal, particularly in human subjects. It is suitable to treat disorders wherein a stimulation or restoration of CD4 T cells is beneficial, such as diseases caused by infectious pathogens or undesirable cell proliferation (e.g., cancers).

### Legend to the figures

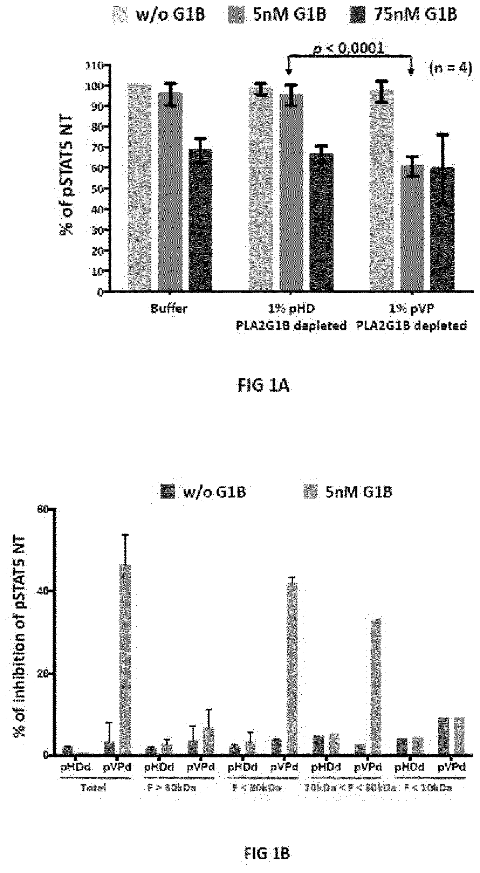

**Figure 1****.** Viremic plasma contains a cofactor that causes sensitivity of CD4 T cells to PLA2-GIB activity. A-CD4 T cells purified from 4 healthy donors were exposed or not (w/o GIB) for 30 min to 5nM or 75nM of PLA2-GIB (GIB) in PBS BSA1% buffer (Buffer), 1% of healthy donor plasma (pHD) or viremic patient plasma (pVP) previously depleted with anti-PLA2-GIB antibody to remove endogenous PLA2-GIB activity on CD4 T cells. Then cells were treated with IL-7 for 15 min and the nuclear translocation of pSTAT5 (pSTAT5 NT) was evaluated by confocal microscopy. Results presented the percentage of pSTAT5 NT normalized with the pSTAT5 NT in response to IL-7 in buffer. Statistical analysis the effect of viremic patient plasma on 5nM of PLA2-GIB was compared to healthy donor plasma using Unpaired t-test. B-Purified CD4 T cells were exposed to 1% of healthy donor plasma (pHD) or viremic patient plasma (pVP) previously depleted with anti-PLA2-GIB antibody and fractionated to separate fraction of molecular weight of more and less than 30kDa and more and less than 10kDa or between 30kDa and 10kDa.

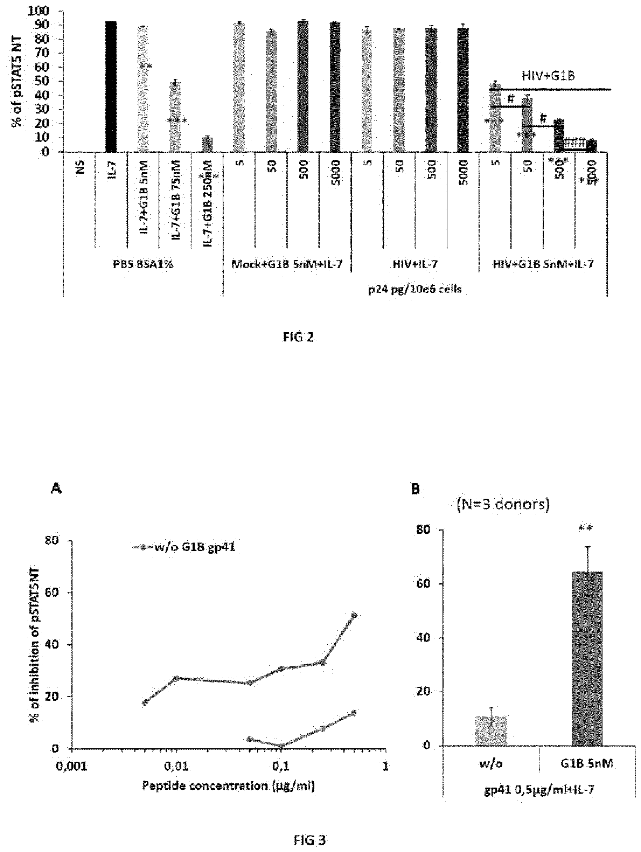

**Figure 2****.** AT-2-inactivated HIV-1 particles cause sensitivity of CD4 T cells to PLA2-GIB activity. Purified CD4 T cells were pretreated for 15 min with PBS BSA 1% buffer, HIV-1 AT-2 inactivated particles or similar dilutions of Mock control. HIV-1 particles were used at 5000, 500, 50 and 5 pg of p24/10e⁶ cells which respectively represents multiplicity of infection (MOI) of 1, 0.1, 0.01 and 0.001. Then cells were treated or not for 30 min with 5nM, 75nM or 250nM of PLA2-GIB in PBS BSA 1% as control of PLA2-GIB inhibition conditions or with 5nM or not of PLA2-GIB with HIV-1 particles or Mock. Then cells were treated with IL-7 for 15min and the nuclear translocation of pSTAT5 (pSTAT5 NT) was evaluated by confocal microscopy. Results are the percentage of pSTAT5 NT in response to IL-7 with the SEM variation calculated on more than 3 independent fields. ** *p*<0.01 and ****p*<0.001 between conditions with GIB relatively to IL-7 treatment without PLA2-GIB. #*p*<0.05, ###*p*<0.001 between conditions with increasing amounts of HIV-1 particles with 5nM of PLA2-GIB. Statistical analyses were performed using unpaired t-test with Welch's correction.
**Figure 3****.** Recombinant gp41 protein causes sensitivity of CD4 T cells to PLA2-GIB inhibitory activity on pSTAT5 NT in response to IL-7. A-Dose-effect of recombinant gp41 protein on PLA2-GIB activity on pSTAT5 NT response to IL-7. Purified CD4 T cells from healthy donor were pretreated for 15min with several amounts of gp41 or buffer (PBS BSA1%), incubated for 30min with 5nM of PLA2-GIB (GIB) or not (w/o GIB) and stimulated with IL-7 for 15min. pSTAT5 NT was analyzed by confocal microscopy. B. Summary of experiments on 3 independent healthy donors of CD4 T cells treated with 0.5µg/ml of gp41 for 15 min, 30 min with 5nM of PLA2-GIB (GIB) or not (w/o GIB) and stimulated with IL-7 for 15 min. A and B, results presented the percentage of inhibition of pSTAT5 NT normalized with the pSTAT5 NT in response to IL-7 in buffer. Statistical analysis of the difference of inhibition with gp41 and 5nM of PLA2-GIB relatively to gp41 alone without PLA2-GIB with unpaired t-test, ** means *p*<0.01.

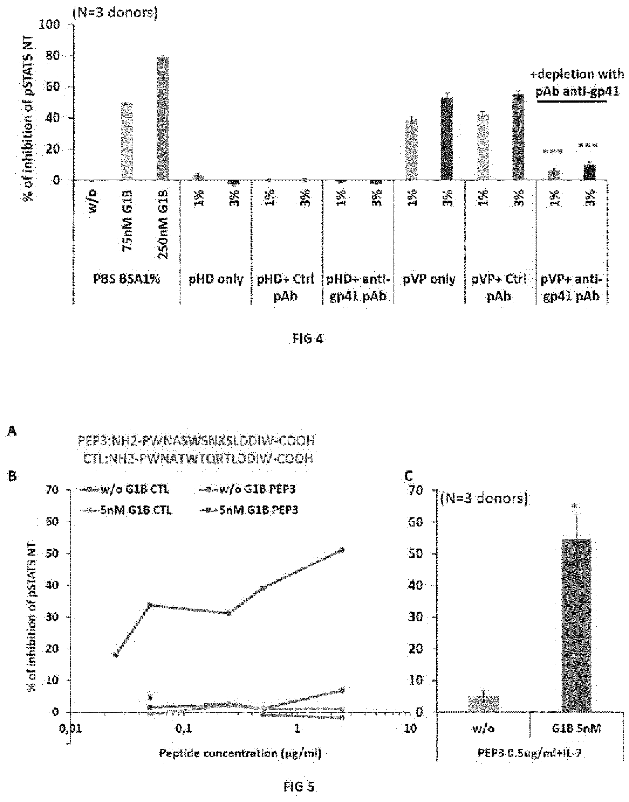

**Figure 4****.** Immunodepletion of viremic patient plasma with anti-gp41 antibody abrogates the inhibitory activity of PLA2G1B on pSTAT5 NT in CD4 T cells (i.e., restores resistance of CD4 T cells to inactivation by PLA2G1B). Purified CD4 T cells from 3 independent healthy donors were treated in 3 independent experiments for 30min with PLA2G1B alone, as positive control of sensitivity to PLA2G1B, healthy donor (HD) plasma or viremic patient (VP) plasma, previously depleted with anti-gp41 polyclonal (pAb anti-gp41), control polyclonal antibody (pAb ctrl) or treated without antibody (only) and stimulated with IL-7 for 15min. Results presented the percentage of inhibition of pSTAT5 NT normalized with the pSTAT5 NT in response to IL-7 in buffer for PLA2G1B or normalized with the same percentage of healthy donor plasma for viremic patient plasma treated samples. *** means that *p*<0.001 with unpaired t-test for the difference of pSTAT5 NT inhibition with pAb ctrl relatively to pAb anti-gp41 treated viremic plasma.
**Figure 5****.** PEP3 peptide induces sensitivity to PLA2G1B inhibitory activity on pSTAT5 NT in CD4 T cells stimulated with IL-7. A-Amino acid sequences of the PEP3 and control (CTL) peptides studied. B-Dose-effect of PEP3 and CTL peptides on PLA2G1B activity on the percentage of inhibition of pSTAT5 NT response to IL-7. Purified CD4 T cells from healthy donor were pretreated for 15min with several amounts of PEP3 or CTL peptides or buffer (PBS BSA1%), incubated for 30min with 5nM of PLA2G1B (5nM G1B) or not (w/o G1B) and stimulated with IL-7 for 15min. pSTAT5 NT was analyzed by confocal microscopy. C-Summary of experiments on 3 independent healthy donors of CD4 T cells treated with 0.5µg/ml of PEP3 for 45min with 5nM of PLA2G1B (G1B 5nM) or not (w/o G1B) and stimulated with IL-7 for 15min. B and C, results presented the percentage of inhibition of pSTAT5 NT normalized with the pSTAT5 NT in response to IL-7 in buffer. Statistical analysis of the difference of inhibition with PEP3 and 5nM of PLA2G1B relatively to PEP3 alone without PLA2G1B with unpaired t-test, * means *p*<0.05.

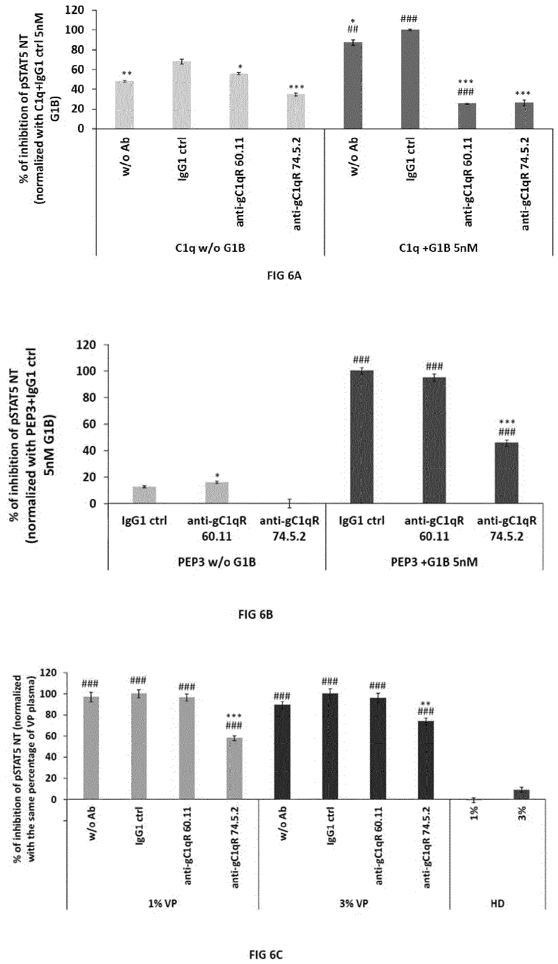

**Figure 6****.** gClqR plays a critical role in the cofactor activity of C1q and PEP3 on PLA2G1B and is involved in viremic patient plasma inhibitory activity. A-Clq has a cofactor activity on PLA2G1B and 60.11 as well as 74.5.2 antibodies against gClqR block C1q PLA2G1B cofactor activity on CD4 T cells. Purified CD4 T cells were preincubated with 60.11, 74.5.2 or mouse control IgG1 (IgGl ctrl) or without antibody (w/o), treated with 10µg/ml of C1q without (w/o) or with 5nM of PLA2G1B (G1B 5nM) and pSTAT5 NT response to IL-7 was analyzed. B-The anti-gClqR 74.5.2 antibody, but not the 60.11 antibody, blocks the PEP3 peptide PLA2G1B cofactor activity on CD4 T cells. Cells were treated as in A with 0.5 µg/ml of PEP3 without (w/o) or with 5nM of PLA2G1B (G1B 5nM). C-The anti-gClqR 74.5.2 antibody, but not the 60.11 antibody, decreases inhibition of pSTAT5 NT in CD4 T cells stimulated with IL-7. Cells were pretreated with anti-gClqR or control antibodies as in A, treated with 1% or 3% viremic patient (pVP) or healthy donor (pHD) plasma for 45 min and pSTAT5 NT response to IL-7 was analyzed. Results in A, B and C are presented as percentage ±SEM of inhibition of pSTAT5 NT normalized with percentage of inhibition with IgG1 ctrl and 5nM G1B with C1q in A or with PEP3 in B and IgG1 ctrl with 1% or 3% of viremic patient plasma in C in one representative experiment. Statistical analyses are the results of unpaired t-test with Welch's correction on at least three independent fields by condition. #*p*<0.05, ##*p*<0.01 and ###*p*<0.001 in each experimental condition with PLA2G1B vs without PLA2G1B in A and B or with each percentage of viremic patient plasma vs with the same percentage of healthy donor plasma in C. **p*<0.05, ***p*<0.01 and ****p*<0.001 in each experimental condition relatively to cells treated with control IgG1 antibody.

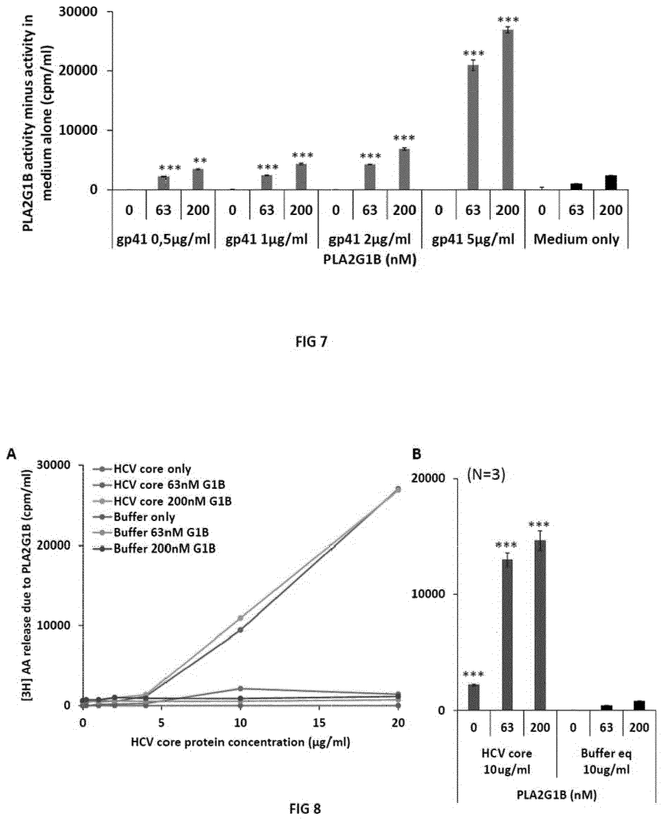

**Figure 7****.** gp41 increases PLA2G1B enzymatic activity on CD4 T cells membranes. Purified CD4 T cells labelled with [3H] arachidonic acid were exposed to several concentrations of recombinant gp41 alone or with 63nM, 200nM of PLA2G1B or with PLA2G1B without gp41 (Medium only). Results are presented as mean cpm/ml ±SEM of triplicate of stimulation due to release of [3H] arachidonic acid by PLA2G1B minus activity in medium alone for each gp41 concentration and are representative of one experiment out of 4 independent experiments with similar results. Statistical analyses are unpaired t-test, *p<0.05, **p<0.01 and ***p<0.001 between experimental condition with gp41 and PLA2G1B vs PLA2G1B alone.
**Figure 8****.** HCV core protein increases PLA2G1B enzymatic activity on CD4 T cells membranes. A-Dose-effect of HCV core protein on [3H] arachidonic acid release and PLA2G1B enzymatic activity. Purified CD4 T cells labelled with [3H] arachidonic acid were exposed to several concentrations of HCV core protein alone (HCV core only) or with 63nM, 200nM of PLA2G1B or with PLA2G1B without HCV core (Buffer only). Results are presented as mean cpm/ml of duplicate of stimulation due to release of [3H] arachidonic acid by PLA2G1B minus activity in medium with buffer alone for each protein concentration of one experiment. B-HCV core protein increases PLA2G1B activity. Purified CD4 T cells labelled with [3H] arachidonic acid were exposed to 10µg/ml of HCV core protein alone (0nM) or with 63nM, 200nM of PLA2G1B or with PLA2G1B without HCV core (Buffer eq 10µg/ml). Results are presented as mean cpm/ml ±SEM of three independent experiments with triplicate of stimulation due to release of [3H] arachidonic acid by PLA2G1B minus activity in medium with buffer alone equivalent to 10µg/ml of HCV core protein. Statistical analyses are unpaired t-test, ***p<0.001 between experimental conditions with HCV core protein alone or with PLA2G1B vs medium alone or PLA2G1B in Buffer, respectively.

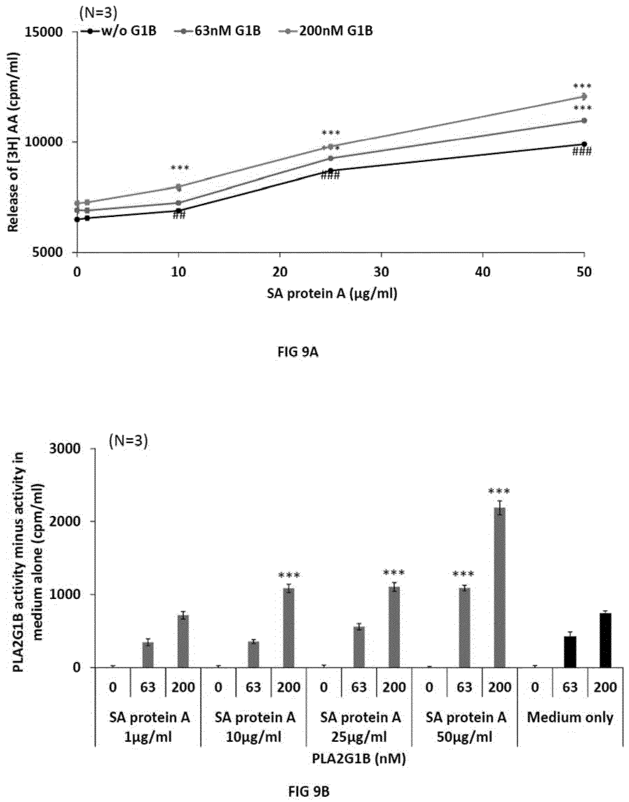

**Figure 9****.** Staphylococcus aureus protein A (SA protein A) increases PLA2G1B enzymatic activity on CD4 T cells membranes. Purified CD4 T cells labelled with [3H] arachidonic acid were exposed to several concentrations of SA protein A alone (w/o G1B) or with 63nM, 200nM of PLA2G1B or with PLA2G1B without SA protein A. A-SA protein A increases basal and PLA2G1B-induced release of [3H] arachidonic acid. Results are presented as mean cpm/ml ± SEM from 3 independent experiments with triplicate of stimulation due to release of [3H] arachidonic acid by SA protein A alone or with PLA2G1B. Statistical analyses are unpaired t-test, ##p<0.01 and ###p<0.001 between experimental conditions with SA protein A alone vs medium alone and *p<0.05, **p<0.01 and ***p<0.001 between experimental conditions with SA protein A with PLA2G1B vs PLA2G1B alone. B-SA protein A increases PLA2G1B activity on CD4 T cells. Results are presented as mean cpm/ml ± SEM due to PLA2G1B activity obtained in 3 independent experiments with triplicate of stimulation with SA protein A and PLA2G1B minus with SA protein A alone or in medium alone. *p<0.05, **p<0.01 and ***p<0.001 between experimental conditions with SA protein A with PLA2G1B vs PLA2G1B alone.

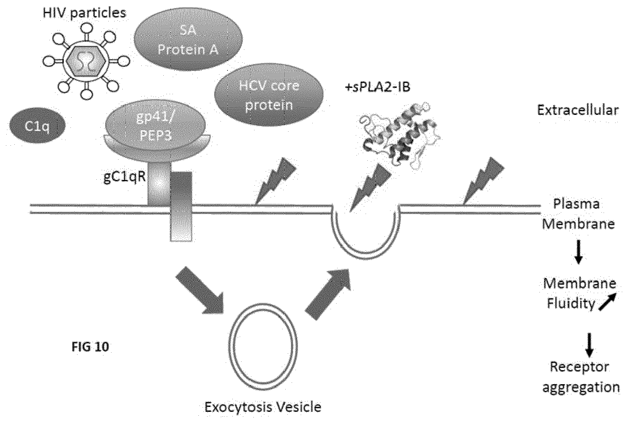

**Figure 10****.** Simplified model of gp41 and other cofactor effect on PLA2G1B activity on CD4 T cells membranes. Binding of PLA2G1B cofactor to gClqR, such as HIV-1 particles, gp41, PEP3, Clq, HCV core or SA protein A, triggers exocytosis of intracellular vesicles. The fusion of these vesicles with plasma membrane changes the lipid composition and causes PLA2G1B activity on CD4 T cells membranes. As a result of PLA2G1B activity, membrane fluidity is increased and cytokines receptors are aggregated in abnormal membrane domain resulting in a dramatic decrease of cytokine signaling and anergy of CD4 T cells.
**Table 1.** Proteins containing a potential gClqR binding element that can act as PLA2-GIB cofactors.
**Table 2.** List of gClqR ligands that can act as PLA2-GIB cofactors.
**Table 3.** Proteins from human pathogens containing a potential gClqR binding element. This table is derived from Table 1 and lists proteins and peptides from human pathogens that can act as PLA2-GIB cofactors, and associated diseases.

### Detailed description of the invention

The invention generally relates to novel therapeutic compositions and methods for treating a mammalian subject in need thereof, which comprise administering a treatment that modulates a PLA2-GIB cofactor. The treatment may comprise administering the cofactor itself; or an activator, agonist or mimotope of the cofactor; or an inhibitor or immunogen of the cofactor. Such treatment is preferably performed in a manner (and the treatment is preferably administered in an amount) which modulates, directly or indirectly, an effect of PLA2-GIB on CD4 T cells, typically in a manner which can maintain or restore resistance of CD4 T cells to inactivation by PLA2-GIB in the subject, or which causes sensitization of CD4 T cells to inactivation by PLA2-GIB in the subject.

### Definitions

As used herein, the term "PLA2-GIB" (or "PLA2-G1B") designates group IB pancreatic phospholipase A2. PLA2-GIB has been identified and cloned from various mammalian species. The human PLA2-GIB protein is disclosed, for instance, in Lambeau and Gelb (2008). The sequence is available on Genbank No. NP_000919.
The amino acid sequence of an exemplary human PLA2-GIB is shown below (SEQ ID NO: 1).

MKLLVLAVLL TVAAADSGIS PRAVWQFRKM IKCVIPGSDP FLEYNNYGCY
CGLGGSGTPV DELDKCCQTH DNCYDQAKKL DSCKFLLDNP YTHTYSYSCS
GSAITCSSKN KECEAFICNC DRNAAICFSK APYNKAHKNL DTKKYCQS

Amino acids 1 to 15 of SEQ ID NO: 1 (underlined) are a signal sequence, and amino acids 16 to 22 of SEQ ID NO: 1 (in bold) are a propeptide sequence.
Within the context of the invention, the term "PLA2-GIB" designates preferably human PLA2-GIB.
The human PLA2-GIB protein may be present under two distinct forms: a pro form (pro-sPLA2-GIB), which is activated by proteolytic cleavage of a pro-peptide, leading to the mature secreted form (sPLA2-GIB). The term PLA2-GIB includes any form of the protein, such as the pro-form and/or the mature form. Typically, the mature secreted form comprises the sequence of amino acid residues 23-148 of SEQ ID NO: 1, or any natural variants thereof.

Natural variants of a protein include variants resulting e.g., from polymorphism or splicing. Natural variants may also include any protein comprising the sequence of SEQ ID NO: 1, or the sequence of amino acid residues 23-148 of SEQ ID NO: 1, with one or more amino acid substitution(s), addition(s) and/or deletion(s) of one or several (typically 1, 2 or 3) amino acid residues. Variants include naturally-occurring variants having e.g., at least 90% amino acid sequence identity to SEQ ID NO: 1. Particular variants contain not more than 10 amino acid substitution(s), addition(s), and/or deletion(s) of one or several (typically 1, 2 or 3) amino acid residues as compared to SEQ ID NO: 1. Typical naturally-occurring variants retain a biological activity of PLA2-GIB. In this regard, in some embodiments, PLA2-GIB has at least one activity selected from induction of formation of membrane microdomains (MMD) in CD4 T cells from healthy subjects, or rendering CD4 T cells of healthy subjects refractory to interleukin signaling, such as refractory to IL-2 signaling or refractory to IL-7 signaling or refractory to IL-4 signaling. In some embodiments rendering CD4 T cells of healthy subjects refractory to interleukin-7 signaling comprises a reduction of STAT5A and/or B phosphorylation in said cells by at least about 10%, at least about 20%, at least about 30%, or at least about 40%. In some embodiments rendering CD4 T cells of healthy subjects refractory to interleukin-7 signaling comprises reducing the rate of nuclear translocation of phospho-STAT5A and/or phospho-STAT5B by at least about 20%, at least about 30%, at least about 40%, or at least about 50%.

The term "sequence identity" as applied to nucleic acid or protein sequences, refers to the quantification (usually percentage) of nucleotide or amino acid residue matches between at least two sequences aligned using a standardized algorithm such as Smith-Waterman alignment (Smith and Waterman (1981) J Mol Biol 147:195-197), CLUSTALW (Thompson et al. (1994) Nucleic Acids Res 22:4673-4680), or BLAST2 (Altschul et al. (1997) Nucleic Acids Res 25:3389-3402). BLAST2 may be used in a standardized and reproducible way to insert gaps in one of the sequences in order to optimize alignment and to achieve a more meaningful comparison between them.

The term "inactivation" indicates, in relation to CD4 T cells, that such cells lose at least part of their ability to contribute to the development of an effective immune response. Inactivation may be partial or complete, transient or permanent. Inactivation designates preferably reducing by at least 10%, 20%, 30%, 40%, 50%, 60%, 70%, 80% or more a function of CD4 T cells, particularly pSTAT5 nuclear translocation and/or CD4 T cell's immunostimulatory activity. Typically, inactive CD4 T cells have no effective pSTAT5 nuclear translocation. In a particular embodiment, an inactive CD4 T cell is an anergic CD4 T cell.

The term "resistance" (or "insensitivity") of CD4 T cells to inactivation by sPLA2-GIB indicates, within the context of this invention, that CD4 T cells are essentially not inactivated in vitro when incubated in the presence of 5nM of sPLA2-GIB. Resistance indicates, for instance, that CD4 T cells retain an active nuclear translocation of pSTAT5 when incubated in vitro in the presence of 5nM sPLA2-GIB and interleukin-7. Resistance (or insensitivity) of CD4 T cells to sPLA2-GIB may also indicate that CD4 T cells incubated in vitro with 5nM PLA2-GIB remain immunologically functional, e.g., do not become anergic.

### Cofactor effect

The inventors have found that many pathogens act by rendering CD4 T cells sensitive to inactivation by PLA2-GIB. Such mechanism is believed to involve the binding of a molecule of (or induced by) the pathogen to gClqR at the surface of CD4 T cells, causing sensitization of CD4 T cells to inactivation by physiological concentrations of PLA2-GIB. In particular, analyzing the mechanism of inactivation of CD4 T cells by PLA2-GIB, the inventors discovered that agonists of gC1qR render CD4 T cells sensitive to low doses of PLA2-GIB. As a result, in the presence of such a cofactor and physiological amounts of PLA2-GIB, CD4 T cells become inactive (e.g., anergic), while they remain active in the presence of physiological amounts of PLA2-GIB only. The inventors verified that gC1q, the natural ligand of gClqR, exhibits such cofactor effect, and that an anti-gC1q antibody can block such cofactor effect. The inventors also surprisingly found that many pathogens, including viruses and cells, actually contain or produce or activate such cofactors that lead to sensitization of CD4 T cells to inactivation by sPLA2-GIB. In particular, the inventors have shown (i) that HCV core protein can bind gClqR and cause sensitization of CD4 T cells to inactivation by sPLA2-GIB, (ii) that Staphylococcus protein A can bind gClqR and cause sensitization of CD4 T cells to inactivation by sPLA2-GIB, and (iii) that HIV gp41 can bind gClqR and cause sensitization of CD4 T cells to inactivation by sPLA2-GIB.

Applicant thus identified a novel general mechanism by which many pathogens induce diseases or pathological status, or (at least temporary) immunodeficiency in mammals, i.e., by producing or activating a cofactor which induces a sensitization of CD4 T cells to inactivation by PLA2-GIB. Such unexpected findings allow applicant to provide novel therapeutic approaches based on the modulation (e.g., blockade or inhibition or stimulation) of said mechanism, thereby preventing, avoiding or at least reducing the pathogenic effects of many pathogens, or inducing an immunosuppression.

It is thus an object of the invention to provide methods and compositions for treating a mammalian subject, comprising administering to the subject a PLA2-GIB cofactor, or a modulator of a PLA2-GIB cofactor.

It is another object of the invention to provide methods and compositions for modulating an immune response in a mammalian subject, comprising administering to the subject a PLA2-GIB cofactor, or a modulator of a PLA2-GIB co-factor.

Another object of the invention relates to a PLA2-GIB cofactor, or a modulator of a PLA2-GIB co-factor, for use for modulating an immune response in a mammalian subject.

Another object of the invention relates to a PLA2-GIB cofactor, or a modulator of a PLA2-GIB co-factor, for use for treating a subject in need thereof, particularly a subject having a disease or condition requiring immunosuppressive or immunostimulating therapy.

It is a further object of the invention to provide methods and compositions for restoring / maintaining resistance of CD4 T cells to inactivation by PLA2-GIB in mammals.

The invention also relates to the use of a PLA2-GIB cofactor, or a modulator of a PLA2-GIB cofactor, for the manufacture of a medicament for treating a subject in need thereof, particularly a subject having a condition requiring immunosuppressive or immunostimulating therapy, more particularly for modulating an immune response in a subject in need thereof.

### PLA2-GIB cofactors

The inventors have surprisingly discovered that many different types of pathogens act as (or produce or activate) a cofactor of PLA2-GIB that, in combination with PLA2-GIB, leads to CD4 T cell inactivation. In particular, as shown Fig 8, HCV core protein causes sensitization of CD4 T cells to inactivation by low concentrations of sPLA2-GIB. Similarly, as shown Fig 9, Staphylococcus protein A causes sensitization of CD4 T cells to inactivation by low concentrations of sPLA2-GIB and, as shown Fig3-7, HIV gp41 causes sensitization of CD4 T cells to inactivation by low concentrations of sPLA2-GIB. The inventors have further discovered that these cofactor molecules are ligands of the gC1qR and that inhibiting their binding to gC1qR also inhibits the cofactor effect (Figures 6B and 6C).

The inventors thus identified various molecules produced by pathogens which can bind gClqR and act as cofactors of sPLA2-GIB.

Within the context of the invention, the term "cofactor" of PLA2-GIB thus designates any molecule which potentiates or amplifies or mediates an effect of PLA2-GIB, particularly an effect of PLA2-GIB on CD4 T cells. Preferred cofactors are molecules which can sensitize CD4 T cells to inactivation by low concentrations of PLA2-GIB.

In a particular embodiment of the invention, the PLA2-GIB cofactor is a ligand of gClqR. The inventors have indeed demonstrated that ligands of gClqR at the surface of CD4 T cells act as cofactors of PLA2-GIB, rendering cells more sensitive to inactivation by PLA2-GIB. More particularly, the PLA2-GIB cofactor is an agonist of gClqR, e.g., can induce signaling through gClqR, more particularly can induce gClqR-mediated exocytosis.

In this respect, the inventors have identified various proteins which can act as cofactor of PLA2-GIB, as listed in Tables 1-3. In a particular embodiment of the invention, the PLA2-GIB cofactor is a protein selected from the proteins of Table 1 or 2, or a gClqR-binding element of such a protein. More particularly, the cofactor may be any protein comprising anyone of SEQ ID NOs: 2-44 or selected from proteins of ID NO: 45-71, more preferably from anyone of SEQ ID NOs: 3, 43, 44 and ID 45-61, even more preferably from anyone of SEQ ID NOs: 3, 43, 44 and ID 45-55, or any fragment or mimotope thereof.

The term "fragment", in relation to such cofactors, designates preferably a fragment containing a gClqR-binding element of such a protein, and/or a fragment retaining a capacity of binding gClqR. Preferred fragments contain at least 5 consecutive amino acid residues, typically between 5 and 100.

In a further particular embodiment, the PLA2-GIB cofactor is a component of a pathogen or a nutrient, preferably a protein or peptide from a pathogen. In a more specific embodiment, the PLA2-GIB cofactor is a viral or bacterial or fungal or parasite protein or peptide. Preferred examples of such cofactors are listed in Tables 2 and 3.

In a specific embodiment, the PLA2-GIB cofactor is HCV core protein, or a fragment or mimotope thereof. In a particular embodiment, the PLA2-GIB cofactor is a protein or peptide comprising or consisting of SEQ ID NO: 43, or a mimotope or fragment thereof.
SEQ ID NO: 43
GenBank: ARQ19013.1

MSTNPKPQRKTKRNTIRRPQDVKFPGGGQIVGGVYLLPRRGPRLGVRATRKTSERSQPRGRRQPIPKARR
PEGRTWAQPGYPWPLYGNEGMGWAGWLLSPRGSRPSWGPTDPRRRSRNLGKVIDTLTCGFADLMGYVPLV
GAPLGGAARALAHGVRALEDGVNYATGNLPGCSFSISLWXLLSCLTIPASA

In another specific embodiment, the PLA2-GIB cofactor is Staphylococcus protein A, or a fragment or mimotope thereof. In a particular embodiment, the PLA2-GIB cofactor is a protein or peptide comprising or consisting of SEQ ID NO: 44, or a mimotope or fragment thereof.
SEQ ID NO :44
NCBI Reference Sequence: YP_498670.1

MKKKNIYSIRKLGVGIASVTLGTLLISGGVTPAANAAQHDEAQQNAFYQVLNMPNLNADQRNGFIQSLKD
DPSQSANVLGEAQKLNDSQAPKADAQQNNFNKDQQSAFYEILNMPNLNEAQRNGFIQSLKDDPSQSTNVL
GEAKKLNESQAPKADNNFNKEQQNAFYEILNMPNLNEEQRNGFIQSLKDDPSQSANLLSEAKKLNESQAP
KADNKFNKEQQNAFYEILHLPNLNEEQRNGFIQSLKDDPSQSANLLAEAKKLNDAQAPKADNKFNKEQQN
AFYEILHLPNLTEEQRNGFIQSLKDDPSVSKEILAEAKKLNDAQAPKEEDNNKPGKEDNNKPGKEDNNKP
GKEDNNKPGKEDNNKPGKEDGNKPGKEDNKKPGKEDGNKPGKEDNKKPGKEDGNKPGKEDGNKPGKEDGN
GVHVVKPGDTVNDIAKANGTTADKIAADNKLADKNMIKPGQELVVDKKQPANHADANKAQALPETGEENP
FIGTTVFGGLSLALGAALLAGRRREL

In another specific embodiment, the PLA2-GIB cofactor is HIV gp41 or rev, or a fragment or mimotope thereof. In a particular embodiment, the PLA2-GIB cofactor is a protein or peptide comprising or consisting of SEQ ID NO: 3 or ID NO: 51, or a fragment or mimotope thereof. Such cofactor is particularly associated with HIV infection.
SEQ ID NO: 3
GenBank reference AAC31817.1

AAIGALFLGFLGAAGSTMGAASVTLTVQARLLLSGIVQQQNNLLRAIESQQHMLRLTVW
GIKQLQARVLAVERYLKDQQLLGFWGCSGKLICTTTVPWNASWSNKSLDDIWNNMTWMQ
WEREIDNYTSLIYSLLEKSQTQQEKNEQELLELDKWASLWNWFDITNWLWYIKIFIMIV
GGLVGLRIVFAVLSIVNRVRQGYSPLSLQTRPPVPRGPDRPEGIEEEGGERDRDTSGRL
VHGFLAIIWVDLRSLFLLSYHHLRDLLLIAARIVELLGRRGWEVLKYWWNLLQYWSQEL
KSSAVSLLNAAAIAVAEGTDRVIEVLQRAGRAILHIPTRIRQGLERALL

In another specific embodiment, the PLA2-GIB cofactor is a protein or peptide comprising or consisting of ID NO: 45, or a fragment or mimotope thereof. Such cofactor is particularly associated with EBV infection.

In another specific embodiment, the PLA2-GIB cofactor is a protein or peptide comprising or consisting of ID NO: 46, or a fragment or mimotope thereof. Such cofactor is particularly associated with Adenovirus infection.

In another specific embodiment, the PLA2-GIB cofactor is a protein or peptide comprising or consisting of ID NO: 47, or a fragment or mimotope thereof. Such cofactor is particularly associated with Hantaan virus infection.

In another specific embodiment, the PLA2-GIB cofactor is a protein or peptide comprising or consisting of ID NO: 48, or a fragment or mimotope thereof. Such cofactor is particularly associated with HSV infection.

In another specific embodiment, the PLA2-GIB cofactor is a protein or peptide comprising or consisting of ID NO: 49 or 50, or a fragment or mimotope thereof. Such cofactor is particularly associated with Rubella virus infection.

In another specific embodiment, the PLA2-GIB cofactor is a protein or peptide comprising or consisting of ID NO: 52, or a fragment or mimotope thereof. Such cofactor is particularly associated with L. monocytogenes infection.

In another specific embodiment, the PLA2-GIB cofactor is a protein or peptide comprising or consisting of ID NO: 53, or a fragment or mimotope thereof. Such cofactor is particularly associated with S. pneumoniae infection.

In another specific embodiment, the PLA2-GIB cofactor is a protein or peptide comprising or consisting of ID NO: 54, or a fragment or mimotope thereof. Such cofactor is particularly associated with B. cereus infection.

In another specific embodiment, the PLA2-GIB cofactor is a protein or peptide comprising or consisting of ID NO: 55, or a fragment or mimotope thereof. Such cofactor is particularly associated with Plasmodium falciparum infection.

In another specific embodiment, the PLA2-GIB cofactor is a protein or peptide comprising SEQ ID NO: 7 or 8, or a fragment or mimotope thereof. Such cofactor is particularly associated with P. gingivalis.

In another specific embodiment, the PLA2-GIB cofactor is a protein or peptide comprising SEQ ID NO: 14, or a fragment or mimotope thereof. Such cofactor is particularly associated with P. mirabilis.

In another specific embodiment, the PLA2-GIB cofactor is a protein or peptide comprising SEQ ID NO: 18, or a fragment or mimotope thereof. Such cofactor is particularly associated with L. weilii str.

In another specific embodiment, the PLA2-GIB cofactor is a protein or peptide comprising SEQ ID NO: 28, or a fragment or mimotope thereof. Such cofactor is particularly associated with T. glycolicus.

In another specific embodiment, the PLA2-GIB cofactor is a protein or peptide comprising SEQ ID NO: 29 or 30, or a fragment or mimotope thereof. Such cofactor is particularly associated with B. fragilis.

In another specific embodiment, the PLA2-GIB cofactor is a protein or peptide comprising SEQ ID NO: 33, or a fragment or mimotope thereof. Such cofactor is particularly associated with C. glabrata.

In another specific embodiment, the PLA2-GIB cofactor is a protein or peptide comprising SEQ ID NO: 38, or a fragment or mimotope thereof. Such cofactor is particularly associated with A. actinomycetemcomitans.

In another specific embodiment, the PLA2-GIB cofactor is a protein or peptide comprising SEQ ID NO: 41, or a fragment or mimotope thereof. Such cofactor is particularly associated with P. somerae.

In another specific embodiment, the PLA2-GIB cofactor is a protein or peptide comprising SEQ ID NO: 42, or a fragment or mimotope thereof. Such cofactor is particularly associated with A. aphrophilus.

### Treatments that modulate the cofactor effect

The invention proposes to treat diseased subjects and/or to restore/enhance CD4 T cell activity in subjects by modulating such a cofactor effect. The invention thus provides methods and compositions for treating diseased subjects and/or for restoring/enhancing CD4 T cell activity in subjects using a PLA2-GIB cofactor or a modulator of a PLA2-GIB cofactor.

The term "modulator" of a PLA2-GIB cofactor designates, within the context of this invention, any molecule which can modulate, directly or indirectly, the expression or activity of a PLA2-GIB cofactor. A modulator may thus be an inhibitor of the PLA2-GIB cofactor; or an immunogen of the PLA2-GIB cofactor (which induces anti-cofactor antibodies); or an activator, agonist or mimotope of the PLA2-GIB cofactor.

### Cofactor inhibitors

The term "inhibitor" of a cofactor designates any molecule or treatment which causes (directly or indirectly) an inhibition of the expression or a function of the cofactor, e.g., cofactor binding to gClqR or cofactor ability to sensitize CD4 T cells to PLA2-GIB. Inhibiting the cofactor designates preferably reducing by at least 10%, 20%, 30%, 40%, 50%, 60%, 70%, 80% or more the expression or a function of the cofactor, as well as completely blocking or suppressing said expression or function. Depending on the situation, the inhibition may be transient, sustained or permanent.

In a particular embodiment, an inhibitor of the cofactor is a gClqR inhibitor. Indeed, cofactors bind gClqR as a target receptor. Blocking or reducing or preventing binding of the cofactor to gClqR using gClqR inhibitors can affect the cofactor effect. The term "gClqR inhibitor" designates any molecule or treatment which causes (directly or indirectly) an inhibition of a function of gClqR, e.g., gClqR-mediated exocytosis.

gC1qR designates the receptor for complement C1q at the surface of cells, particularly of CD4 T cells, especially the human form of said receptor. gC1qR is also known as C1q binding protein (C1QBP), ASF/SF2-associated protein p32 (SF2P32); Glycoprotein gC1qBP; Hyaluronan-binding protein 1 (HABP1); Mitochondrial matrix protein p32; gC1q-R protein; p33; C1qBP and GC1QBP. The amino acid sequence of the receptor was disclosed in the art. An exemplary amino acid sequence of human gClqR is reproduced below (SEQ ID NO: 2):

MLPLLRCVPRVLGSSVAGLRAAAPASPFRQLLQPAPRLCTRPFGLLSVRAGSER
RPGLLRPRGPCACGCGCGSLHTDGDKAFVDFLSDEIKEERKIQKHKTLPKMSGG
WELELNGTEAKLVRKVAGEKITVTFNINNSIPPTFDGEEEPSQGQKVEEQEPELT
STPNFVVEVIKNDDGKKALVLDCHYPEDEVGQEDEAESDIFSIREVSFQSTGESE
WKDTNYTLNTDSLDWALYDHLMDFLADRGVDNTFADELVELSTALEHQEYIT
FLEDLKSFVKSQ

The term gClqR designates any receptor of SEQ ID NO: 2 (accession number UniProtKB/Swiss-Prot: Q07021.1) above, as well as processed forms and variants thereof. Variants include naturally-occurring variants having e.g., at least 90% amino acid sequence identity to SEQ ID NO: 2.

Upon binding of a cofactor, gClqR triggers a signaling pathway that results in exocytosis of intracellular vesicles. Without being bound by theory, it is believed that the fusion of these vesicles with the cytoplasmic membrane could change the lipid composition and increase sPLA2-GIB activity on CD4 T cells membrane, resulting in an inhibition of phosphoSTAT5 signaling (see Fig 10). In particular, the fusion of these vesicles with plasma membrane can change the lipid composition and cause sPLA2-GIB activity on CD4 T cells membranes. As a result, membrane fluidity is increased and cytokines receptors are aggregated in abnormal membrane domain, resulting in a dramatic decrease of cytokine signaling, and anergy of CD4 T cells.

The term gClqR inhibitor thus includes any molecule which binds to gClqR, or to a partner of gC1qR, and inhibits a function of gC1qR, such as gC1qR-mediated exocytosis.

In another embodiment, the cofactor inhibitor is a molecule which directly inhibits an activity of the cofactor, e.g., which binds the cofactor and/or inhibits binding of the cofactor to its receptor.

Preferred examples of cofactor inhibitors include, for instance, antibodies and variants thereof, synthetic specific ligands, peptides, small drugs, or inhibitory nucleic acids.

### Antibodies

In a first embodiment, a cofactor inhibitor is an antibody or an antibody variant/fragment having essentially the same antigen specificity, or a nucleic acid encoding such an antibody or variant/fragment. The antibody may bind a cofactor, or gClqR, or a partner of gClqR, or a gClqR-binding element thereof, and preferably inhibits a function of the cognate antigen (e.g., gClqR or the cofactor).

Antibodies can be synthetic, monoclonal, or polyclonal and can be made by techniques well known per se in the art.

The term "antibodies" is meant to include polyclonal antibodies, monoclonal antibodies, fragments thereof, such as F(ab')2 and Fab fragments, single-chain variable fragments (scFvs), single-domain antibody fragments (VHHs or Nanobodies), bivalent antibody fragments (diabodies), as well as any recombinantly and synthetically produced binding partners, human antibodies or humanized antibodies.

Antibodies are defined to be specifically binding, preferably if they bind to the cognate antigen with a Ka of greater than or equal to about 10⁷ M-1. Affinities of antibodies can be readily determined using conventional techniques, for example those described by Scatchard et al., Ann. N.Y. Acad. Sci., 51:660 (1949).

Polyclonal antibodies can be readily generated from a variety of sources, for example, horses, cows, donkeys, goats, sheeps, dogs, chickens, rabbits, mice, hamsters, or rats, using procedures that are well known in the art. In general, a purified immunogen, optionally appropriately conjugated, is administered to the host animal typically through parenteral injection. The immunogenicity of immunogen can be enhanced through the use of an adjuvant, for example, Freund's complete or incomplete adjuvant. Following booster immunizations, small samples of serum are collected and tested for reactivity to the antigen polypeptide. Examples of various assays useful for such determination include those described in Antibodies: A Laboratory Manual, Harlow and Lane (eds.), Cold Spring Harbor Laboratory Press, 1988; as well as procedures, such as countercurrent immuno-electrophoresis (CIEP), radioimmunoassay, radio-immunoprecipitation, enzyme-linked immunosorbent assays (ELISA), dot blot assays, and sandwich assays. See U.S. Pat. Nos. 4,376,110 and 4,486,530.

Monoclonal antibodies can be readily prepared using well known procedures. See, for example, the procedures described in U.S. Pat. Nos. RE 32,011, 4,902,614, 4,543,439, and 4,411,993; Monoclonal Antibodies, Hybridomas: A New Dimension in Biological Analyses, Plenum Press, Kennett, McKeam, and Bechtol (eds.), 1980. For example, the host animals, such as mice, can be injected intraperitoneally at least once and preferably at least twice at about 3 week intervals with isolated and purified immunogen, optionally in the presence of adjuvant. Mouse sera are then assayed by conventional dot blot technique or antibody capture (ABC) to determine which animal is best to fuse. Approximately two to three weeks later, the mice are given an intravenous boost of protein or peptide. Mice are later sacrificed and spleen cells fused with commercially available myeloma cells, such as Ag8.653 (ATCC), following established protocols. Briefly, the myeloma cells are washed several times in media and fused to mouse spleen cells at a ratio of about three spleen cells to one myeloma cell. The fusing agent can be any suitable agent used in the art, for example, polyethylene glycol (PEG). Fusion is plated out into plates containing media that allows for the selective growth of the fused cells. The fused cells can then be allowed to grow for approximately eight days. Supernatants from resultant hybridomas are collected and added to a plate that is first coated with goat anti-mouse Ig. Following washes, a label is added to each well followed by incubation. Positive wells can be subsequently detected. Positive clones can be grown in bulk culture and supernatants are subsequently purified over a Protein A column (Pharmacia). Monoclonal antibodies may also be produced using alternative techniques, such as those described by Alting-Mees et al., "Monoclonal Antibody Expression Libraries: A Rapid Alternative to Hybridomas", Strategies in Molecular Biology 3:1-9 (1990), which is incorporated herein by reference. Similarly, binding partners can be constructed using recombinant DNA techniques to incorporate the variable regions of a gene that encodes a specific binding antibody. Such a technique is described in Larrick et al., Biotechnology, 7:394 (1989).

Antigen-binding fragments of antibodies, which can be produced by conventional techniques, are also encompassed by the present invention. Examples of such fragments include, but are not limited to, Fab and F(ab')2 fragments. Antibody fragments and derivatives produced by genetic engineering techniques are also provided.

The monoclonal antibodies of the invention also include chimeric antibodies, e.g., humanized versions of murine monoclonal antibodies. Such humanized antibodies can be prepared by known techniques, and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment can comprise the antigen binding site of a murine monoclonal antibody and a variable region fragment (lacking the antigen-binding site) derived from a human antibody. Procedures for the production of chimeric and further engineered monoclonal antibodies include those described in Riechmann et al. (Nature 332:323, 1988), Liu et al. (PNAS 84:3439, 1987), Larrick et al. (Bio/Technology 7:934, 1989), and Winter and Harris (TIPS 14:139, May, 1993). Procedures to generate antibodies transgenically can be found in GB 2,272,440, U.S. Pat. Nos. 5,569,825 and 5,545,806. Antibodies produced by genetic engineering methods, such as chimeric and humanized monoclonal antibodies, comprising both human and non-human portions, which can be made using standard recombinant DNA techniques, can be used. Such chimeric and humanized monoclonal antibodies can be produced by genetic engineering using standard DNA techniques known in the art, for example using methods described in Robinson et al. International Publication No. WO 87/02671; Akira, et al. European Patent Application 0184187; Taniguchi, M., European Patent Application 0171496; Morrison et al. European Patent Application 0173494; Neuberger et al. PCT International Publication No. WO 86/01533; Cabilly et al. U.S. Pat. No. 4,816,567; Cabilly et al. European Patent Application 0125023; Better et al., Science 240:1041 1043, 1988; Liu et al., PNAS 84:3439 3443, 1987; Liu et al., J. Immunol. 139:3521 3526, 1987; Sun et al. PNAS 84:214 218, 1987; Nishimura et al., Canc. Res. 47:999 1005, 1987; Wood et al., Nature 314:446 449, 1985; and Shaw et al., J. Natl. Cancer Inst. 80:1553 1559, 1988); Morrison, S. L., Science 229:1202 1207, 1985; Oi et al., BioTechniques 4:214, 1986; Winter U.S. Pat. No. 5,225,539; Jones et al., Nature 321:552 525, 1986; Verhoeyan et al., Science 239:1534, 1988; and Beidler et al., J. Immunol. 141:4053 4060, 1988.

In connection with synthetic and semi-synthetic antibodies, such terms are intended to cover but are not limited to antibody fragments, isotype switched antibodies, humanized antibodies (e.g., mouse-human, human-mouse), hybrids, antibodies having plural specificities, and fully synthetic antibody-like molecules.

Human monoclonal antibodies can also be prepared by constructing a combinatorial immunoglobulin library, such as a Fab phage display library or a scFv phage display library, using immunoglobulin light chain and heavy chain cDNAs prepared from mRNA derived from lymphocytes of a subject. See, e.g., McCafferty et al. PCT publication WO 92/01047; Marks et al. (1991) J. Mol. Biol. 222:581 597; and Griffths et al. (1993) EMBO J 12:725 734. In addition, a combinatorial library of antibody variable regions can be generated by mutating a known human antibody. For example, a variable region of a human antibody known to bind gClqR can be mutated by, for example, using randomly altered mutagenized oligonucleotides, to generate a library of mutated variable regions which can then be screened to bind to gC1qR. Methods of inducing random mutagenesis within the CDR regions of immunoglobin heavy and/or light chains, methods of crossing randomized heavy and light chains to form pairings and screening methods can be found in, for example, Barbas et al. PCT publication WO 96/07754; Barbas et al. (1992) Proc. Nat'1 Acad. Sci. USA 89:4457 4461.

Antibodies of the invention may be directed against gClqR, a gClqR ligand, or a C1qR partner, and cause an inhibition of signaling mediated by gClqR. For preparing antibodies of the invention, an immunogen may be used comprising gClqR, a gClqR ligand, or a gC1qR partner, or a fragment, variant, or fusion molecule thereof.

### Antibodies to gC1qR

Particular antibodies of the invention bind a gClqR epitope, and/or have been generated by immunization with a polypeptide comprising a gClqR epitope, selected from the mature gC1qR protein or a fragment of gC1qR comprising at least 8 consecutive amino acid residues thereof. Preferred anti-gClqR antibodies of the invention bind an epitope of a ligand-binding site within gClqR, thereby interfering with binding of the ligand. In a particular embodiment, the antibodies bind an epitope comprised between amino acid residues 76-282 of SEQ ID NO: 2, which contain the gClqR ligand bind site. C1q binding to gClqR can involve at least three different motifs on gClqR, namely: amino acid residues 75-96, 190-202 and 144-162 (by reference to SEQ ID NO: 2). HCV core protein binding to gClqR can involve at least two different motifs on gClqR, namely: amino acid residues 144-148 and 196-202 (by reference to SEQ ID NO: 2). HIV gp41 binding to gClqR can involve at least amino acid residues 174-180 on gClqR (by reference to SEQ ID NO: 2).

It is thus preferred to use an antibody (or variant thereof) which binds an epitope containing at least one amino acid residue contained in one of said epitopes or close to one of said epitopes. Examples of such antibodies include antibody 60.11, which binds to residues 75-96 of gClqR; as well as antibody 74.5.2, which binds to an epitope with the residues 204 to 218.

Preferred gC1qR inhibitors are therefore monoclonal antibodies against gC1qR, more preferably against an epitope of gClqR located within amino acid residues 76-282 of the protein (by reference to SEQ ID NO: 2), even more preferably an epitope containing an amino acid residue selected from amino acids 75-96, 144-162, 174-180, and 190-210.

Preferred antibodies are neutralizing (or antagonist) antibodies, i.e., they prevent or inhibit or reduce binding of a natural ligand to the receptor and/or signaling through the receptor.

### Antibodies to a PLA2-GIB cofactor

Other particular inhibitors of the invention are antibodies that bind a PLA2-GIB cofactor and/or have been generated by immunization with a PLA2-GIB cofactor or a fragment thereof, and preferably inhibit at least partially binding of such a cofactor to gC1qR.

Particular antibodies of the invention are polyclonal antibodies or monoclonal antibodies, or variants thereof, which bind a protein selected from the proteins listed in Tables 1 and 2, and inhibit at least partially the binding of said protein to gC1qR. Preferred antibodies of the invention are polyclonal antibodies or monoclonal antibodies, or variants thereof, which bind a protein selected from the proteins listed in Tables 2 and 3, and inhibit at least partially the binding of said protein to gClqR, even more particularly a protein selected from the proteins listed in Table 2, and inhibit at least partially the binding of said protein to gC1qR.

In a particular embodiment, the C1qR inhibitor is an antibody or a variant thereof that binds a protein selected from SEQ ID NOs: 2-44 and ID NO: 45-71, more preferably from SEQ ID NOs: 2, 3, 43, 44 and from ID NO: 45-61, even more preferably from SEQ ID NOs: 3, 43, 44 and ID NO: 45-55, and inhibits at least partially the binding of said protein to gClqR.

Particular antibodies or variants of the invention bind an epitope within the C1qR ligand contained in (or overlapping with) the gClqR-binding element or domain of said ligand, typically comprising at least 1 amino acid residue of said ligand that is involved in the binding of said ligand to gC1qR.

In a particular embodiment, the inhibitor is an antibody or variant thereof which binds a protein or peptide comprising SEQ ID NO: 7 or 8. Such inhibitor is particularly useful for treating disorders associated with P. gingivalis, such as Pancreatic cancer, Chronic periodontal disease, Rheumatoid polyarthritis, or Alzheimer's Disease.

In a particular embodiment, the inhibitor is an antibody or variant thereof which binds a protein or peptide comprising SEQ ID NO: 14. Such inhibitor is particularly useful for treating disorders associated with P. mirabilis, such as Urinary tract infections or Osteomyelitis in an HIV-patient.

In a particular embodiment, the inhibitor is an antibody or variant thereof which binds a protein or peptide comprising SEQ ID NO: 18. Such inhibitor is particularly useful for treating disorders associated with L. weilii str., such as leptospirosis.

In a particular embodiment, the inhibitor is an antibody or variant thereof which binds a protein or peptide comprising SEQ ID NO: 28. Such inhibitor is particularly useful for treating disorders associated with T. glycolicus, such as wounds infections.

In a particular embodiment, the inhibitor is an antibody or variant thereof which binds a protein or peptide comprising SEQ ID NO: 29 or 30. Such inhibitor is particularly useful for treating disorders associated with B. fragilis, such as Peritoneal infections, bacteremia, subcutaneous abscesses or burns.

In a particular embodiment, the inhibitor is an antibody or variant thereof which binds a protein or peptide comprising SEQ ID NO: 33. Such inhibitor is particularly useful for treating disorders associated with C. glabrata, such as Cutaneous candidiasis in HIV/AIDS, cancer and organ transplantation.

In a particular embodiment, the inhibitor is an antibody or variant thereof which binds a protein or peptide comprising SEQ ID NO: 38. Such inhibitor is particularly useful for treating disorders associated with A. actinomycetemcomitans, such as Agressive Periodontitis, Bacterial vaginosis, Endocarditis, Actinomycosis, or Rheumatoid arthritis.

In a particular embodiment, the inhibitor is an antibody or variant thereof which binds a protein or peptide comprising SEQ ID NO: 41. Such inhibitor is particularly useful for treating disorders associated with P. somerae, such as Chronic skin, soft tissue and bone infections.

In a particular embodiment, the inhibitor is an antibody or variant thereof which binds a protein or peptide comprising SEQ ID NO: 42. Such inhibitor is particularly useful for treating disorders associated with A. aphrophilus, such as Endocarditis, brain abscesses, vertebral osteomyelitis and bacteremia.

In a particular embodiment, the inhibitor is an antibody or variant thereof which binds a protein or peptide containing or consisting of SEQ ID NO: 3 or ID45. Such inhibitor is particularly useful for treating HIV infection.

In a particular embodiment, the inhibitor is an antibody or variant thereof which binds a protein or peptide containing or consisting of SEQ ID NO: 43. Such inhibitor is particularly useful for treating HCV infection.

In a particular embodiment, the inhibitor is an antibody or variant thereof which binds a protein or peptide containing or consisting of ID NO: 51. Such inhibitor is particularly useful for treating EBV infection.

In a particular embodiment, the inhibitor is an antibody or variant thereof which binds a protein or peptide containing or consisting of ID NO: 46. Such inhibitor is particularly useful for treating Adenovirus infection.

In a particular embodiment, the inhibitor is an antibody or variant thereof which binds a protein or peptide containing or consisting of ID NO: 47. Such inhibitor is particularly useful for treating HTNV infection.

In a particular embodiment, the inhibitor is an antibody or variant thereof which binds a protein or peptide containing or consisting of ID NO: 48. Such inhibitor is particularly useful for treating HSV infection.

In a particular embodiment, the inhibitor is an antibody or variant thereof which binds a protein or peptide containing or consisting of ID NO: 49 or 50. Such inhibitor is particularly useful for treating Rubella virus infection.

In a particular embodiment, the inhibitor is an antibody or variant thereof which binds a protein or peptide containing or consisting of SEQ ID NO: 44. Such inhibitor is particularly useful for treating Staphylococcus aureus infection.

In a particular embodiment, the inhibitor is an antibody or variant thereof which binds a protein or peptide containing or consisting of ID NO: 52. Such inhibitor is particularly useful for treating L. monocytogenes infection.

In a particular embodiment, the inhibitor is an antibody or variant thereof which binds a protein or peptide containing or consisting of ID NO: 53. Such inhibitor is particularly useful for treating S. pneumoniae infection.

In a particular embodiment, the inhibitor is an antibody or variant thereof which binds a protein or peptide containing or consisting of ID NO: 54. Such inhibitor is particularly useful for treating B. cereus infection.

In a particular embodiment, the inhibitor is an antibody or variant thereof which binds a protein or peptide containing or consisting of ID NO: 55. Such inhibitor is particularly useful for treating P. falciparum infection.

### Inhibitory Nucleic acids

In an alternative embodiment, the cofactor inhibitor is an inhibitory nucleic acid. Preferred inhibitory nucleic acids include aptamers which are designed to bind the cofactor, or gClqR, or a partner of gClqR, and to inhibit a function thereof.

### Peptides

In an alternative embodiment, the cofactor inhibitor is a peptide that inhibits a function of the cofactor. The peptide is typically a molecule that selectively binds a cofactor, a gClqR, or a partner of gClqR.

Peptides preferably contain from 4 to 30 amino acid residues, and their sequence may be identical to a domain of gClqR or to a domain of a cofactor (bait peptide), or their sequence may contain a variation as compared to the sequence of a domain of gClqR or to a domain of a cofactor (peptide antagonist).

Preferred peptides of the invention contain from 4 to 30 consecutive amino acid residues of SEQ ID NO: 2 (gClqR) or of a cofactor selected from anyone of SEQ ID NOs: 3-71, and may contain at least 1 modification.

Examples of such peptides include, for instance the peptide with amino acid residues 144-162 of SEQ ID NO: 2 (gClqR) and the peptide with amino acid residues 204-218 of SEQ ID NO: 2 (gClqR).

The modification may consist of an amino acid substitution. The modification may alternatively (or in addition) consist of a chemical modification, such as addition of a chemical group to one (or both) ends of the peptide, or to a lateral chain thereof, or to a peptide bond. In this regard, the peptides of the invention can comprise peptide, non-peptide and/or modified peptide bonds. In a particular embodiment, the peptides comprise at least one peptidomimetic bond selected from intercalation of a methylene (-CH₂-) or phosphate (-PO₂-) group, secondary amine (-NH-) or oxygen (-O-), alpha-azapeptides, alpha-alkylpeptides, N-alkylpeptides, phosphonamidates, depsipeptides, hydroxymethylenes, hydroxyethylenes, dihydroxyethylenes, hydroxyethylamines, retro-inverso peptides, methyleneoxy, cetomethylene, esters, phosphinates, phosphinics, or phosphonamides. Also, the peptides may comprise a protected N-ter and/or C-ter function, for example, by acylation, and/or amidation and/or esterification.

The peptides of the invention may be produced by techniques known per se in the art such as chemical, biological, and/or genetic synthesis.

Each of these peptides, in isolated form, represents a particular object of the present invention. The term "isolated", as used herein, refers to molecules (e.g., nucleic or amino acid) that are removed from a component of their natural environment, isolated or separated, and are at least 60% free, preferably 75% free, and most preferably 90% free from other components with which they are naturally associated. An "isolated" polypeptide (or protein) is for instance a polypeptide separated from a component of its natural environment and, preferably purified to greater than 90% or 95% purity as determined by, for example, electrophoretic (e.g., SDS-PAGE, isoelectric focusing (IEF), capillary electrophoresis) or chromatographic (e.g., ion exchange or reverse phase HPLC) migration. An "isolated" nucleic acid refers to a nucleic acid molecule separated from a component of its natural environment and/or assembled in a different construct (e.g., a vector, expression cassette, recombinant host, etc.).

### Small drugs

Preferred small drugs are hydrocarbon compounds that selectively bind gC1qR or a cofactor.

Small drugs are preferably obtainable by a method comprising: (i) contacting a test compound with a cell expressing gClqR, (ii) selecting a test compound which binds gClqR, and (iii) selecting a compound of (ii) which inhibits an activity of gClqR. Such a method represents a particular object of the invention.

### gC1qR soluble receptors

In an alternative embodiment, the cofactor inhibitor is a soluble form of gClqR.

### Immunogens

In an alternative (or cumulative) embodiment, inhibition of the cofactor in a subject is obtained by using (e.g., vaccinating or immunizing the subject with) an immunogen of the cofactor. As a result, the subject produces antibodies (or cells) which inhibit the cofactor. In particular, administration(s) of a cofactor immunogen (e.g., any immunogenic portion of a cofactor) can generate antibodies in the treated subject. These antibodies will inhibit the cofactor effect as immunotherapy or a vaccine prophylaxy.

An object of the invention thus resides in a method of vaccinating a subject comprising administering to the subject an immunogen of a PLA2-GIB cofactor.

A further object of the invention relates to an immunogen of a PLA2-GIB cofactor for use to vaccinate a subject in need thereof.

In a particular embodiment, the immunogen of a PLA2-GIB cofactor antigen used for vaccination is an inactivated immunogenic molecule that induces an immune response against the cofactor in a subject. Inactivation may be obtained e.g., by chemically or physically altering the cofactor or by mutating or truncating the protein, or both; and immunogenicity may be obtained as a result of the inactivation and/or by further conjugating the protein to a suitable carrier or hapten, such as KLH, HSA, polylysine, a viral anatoxin, or the like, and/or by polymerization, or the like. The immunogen may thus be chemically or physically modified, e.g., to improve its immunogenicity.

In a preferred embodiment, the immunogen of a PLA2-GIB cofactor of the invention comprises the entire cofactor.

In an alternative embodiment, the immunogen of a PLA2-GIB cofactor comprises a fragment of a cofactor comprising at least 6 consecutive amino acid residues and containing an immunogenic epitope thereof. In a preferred embodiment, the immunogen comprises at least from 6 to 20 amino acid residues. Preferred immunogens of the invention comprise or consist of from 4 to 30 consecutive amino acid residues of a protein selected from anyone of SEQ ID NOs: 2-44 and ID NO: 45-71 (or of a corresponding sequence of a natural variant).

The immunogen may be in various forms such as in free form, polymerized, chemically or physically modified, and/or coupled (i.e., linked) to a carrier molecule. Coupling to a carrier may increase the immunogenicity and (further) suppress the biological activity of the immunogen. In this regard, the carrier molecule may be any carrier molecule or protein conventionally used in immunology such as for instance KLH (Keyhole limpet hemocyanin), ovalbumin, bovine serum albumin (BSA), a viral or bacterial anatoxin such as toxoid tetanos, toxoid diphteric B cholera toxin, mutants thereof such as diphtheria toxin CRM 197, an outer membrane vesicle protein, a polylysine molecule, or a virus like particle (VLP). In a preferred embodiment, the carrier is KLH or CRM197 or a VLP.

Coupling of the immunogen to a carrier may be performed by covalent chemistry using linking chemical groups or reactions, such as for instance glutaraldehyde, biotin, etc. Preferably, the conjugate or the immunogen is submitted to treatment with formaldehyde in order to complete inactivation of the cofactor.

The immunogenicity of the immunogen may be tested by various methods, such as by immunization of a non-human animal grafted with human immune cells, followed by verification of the presence of antibodies, or by sandwich ELISA using human or humanized antibodies. The lack of biological activity may be verified by any of the activity tests described in the application.

In a particular embodiment, the invention relates to an inactivated and immunogenic PLA2-GIB cofactor.

In a further particular embodiment, the invention relates to a PLA2-GIB cofactor protein or a fragment thereof conjugated to a carrier molecule, preferably to KLH.

In a further aspect, the invention relates to a vaccine comprising an immunogen of PLA2-GIB cofactor, a suitable excipient and, optionally, a suitable adjuvant.

A further object of the invention relates to a method for inducing the production of antibodies that neutralize the activity of a PLA2-GIB cofactor in a subject in need thereof, the method comprising administering to said subject an effective amount of a immunogen or vaccine as defined above.

Administration of an immunogen or vaccine of the invention may be by any suitable route, such as by injection, preferably intramuscular, subcutaneous, transdermal, intraveinous or intraarterial; by nasal, oral, mucosal or rectal administration.

### Activators, agonists and mimotopes

The term cofactor "agonist", within the context of the present invention, encompasses any substance having an activity of the cofactor, such as an ability to sensitize CD4 T cells to PLA2-GIB. agonists of the cofactor may include agonists of gC1qR. An example of an agonist is the cofactor itself.

The term "activator" of a cofactor designates, within the context of the present invention, any substance mediating or up-regulating the cofactor expression or activity such as, without limitation, a peptide, a polypeptide, a recombinant protein, a conjugate, a natural or artificial ligand, a degradation product, a homolog, a nucleic acid, DNA, RNA, an aptamer, etc., or a combination thereof.

Mimotopes include any synthetic molecule which can reproduce an activity of the cofactor.

In a particular embodiment, the invention relates to methods for inhibiting an immune response in a subject, comprising administering to the subject an agonist or activator or mimotope of a PLA2-GIB cofactor, such a cofactor itself, or a nucleic acid encoding the cofactor.

### Compositions & methods of treatment

The invention also relates to a composition comprising a cofactor or modulator as defined above and, preferably, a pharmaceutically acceptable diluent, excipient or carrier.

A "pharmaceutical composition" refers to a formulation of a compound of the invention (active ingredient) and a medium generally accepted in the art for the delivery of biologically active compounds to the subject in need thereof. Such a carrier includes all pharmaceutically acceptable carriers, diluents, medium or supports therefore. Conventional pharmaceutical practice may be employed to provide suitable formulations or compositions to subjects, for example in unit dosage form.

The compounds or compositions according to the invention may be formulated in the form of ointment, gel, paste, liquid solutions, suspensions, tablets, gelatin capsules, capsules, suppository, powders, nasal drops, or aerosol, preferably in the form of an injectable solution or suspension. For injections, the compounds are generally packaged in the form of liquid suspensions, which may be injected via syringes or perfusions, for example. In this respect, the compounds are generally dissolved in saline, physiological, isotonic or buffered solutions, compatible with pharmaceutical use and known to the person skilled in the art. Thus, the compositions may contain one or more agents or excipients selected from dispersants, solubilizers, stabilizers, preservatives, etc. Agents or excipients that can be used in liquid and/or injectable formulations are notably methylcellulose, hydroxymethylcellulose, carboxymethylcellulose, polysorbate 80, mannitol, gelatin, lactose, vegetable oils, acacia, etc. The carrier can also be selected for example from methyl-beta-cyclodextrin, a polymer of acrylic acid (such as carbopol), a mixture of polyethylene glycol and polypropylene glycol, monoethanolamine and hydroxymethyl cellulose.

The compositions generally comprise an effective amount of a compound of the invention, e.g., an amount that is effective to modulate directly or indirectly an effect of PLA2-GIB on CD4 T cells. Inhibitors are typically used in an amount effective to maintain/restore resistance of CD4 T cells to inactivation by PLA2-GIB. Generally, the compositions according to the invention comprise from about 1 µg to 1000 mg of a cofactor or modulator, such as from 0.001-0.01, 0.01-0.1, 0.05-100, 0.05-10, 0.05-5, 0.05-1, 0.1-100, 0.1-1.0, 0.1-5, 1.0-10, 5-10, 10-20, 20-50, and 50-100 mg, for example between 0.05 and 100 mg, preferably between 0.05 and 5 mg, for example 0.05, 0.1, 0.2, 0.3, 0.4, 0.5, 1, 2, 3, 4 or 5 mg. The dosage may be adjusted by the skilled person depending on the agent and the disease.

The compositions of the invention can further comprise one or more additional active compounds, for simultaneous or sequential use. Examples of additional active compounds include, but are not limited to, chemotherapeutic drug, antibiotics, antiparasitic agents, antifungal agents or antiviral agents.

Examples of antibiotics include, but are not limited to, amoxicillin, clarithromycin, cefuroxime, cephalexin ciprofloxacin, doxycycline, metronidazole, terbinafine, levofloxacin, nitrofurantoin, tetracycline, and azithromycin.

Examples of anti-fungal agents, include, but are not limited to, clotrimazole, butenafine, butoconazole, ciclopirox, clioquinol, clioquinol, clotrimazole, econazole, fluconazole, flucytosine, griseofulvin, haloprogin, itraconazole, ketoconazole, miconazole, naftifine, nystatin, oxiconazole, sulconazole, terbinafine, terconazole, tioconazole, and tolnaftate. Examples of anti-viral agents, include, but are not limited to, zidovudine, didanosine, zalcitabine, stavudine, lamivudine, abacavir, tenofovir, nevirapine, delavirdine, efavirenz, saquinavir, ritonavir, indinavir, nelfinavir, saquinavir, amprenavir, and lopinavir.

Examples of other active drugs include any sPLA2-GIB modulator, as disclosed for instance in WO2015/097140, in WO2017/037041 or in WO2017/060405.

The invention also relates to a method for preparing a pharmaceutical composition, comprising mixing a cofactor or modulator as previously described and a pharmaceutically acceptable diluent or excipient, and formulating the composition in any suitable form or container (syringe, ampoule, flask, bottle, pouch, etc.).

The invention also relates to a kit comprising (i) a composition comprising a cofactor or modulator as previously described, (ii) at least one container, and optionally (iii) written instructions for using the kit.

Because various different types of pathogens act through the novel mechanism identified by the inventors, the compounds and compositions of the invention may be used to treat a variety of diseases, such as infectious diseases and diseases related to an inappropriate (e.g., defective or improper) immune response, particularly to an inappropriate CD4 T cell activity, as well as any disease where an increased immunity may ameliorate the subject condition. These diseases are sometime referred to as "immune disorders" in the present application. This includes immunodefective situations (e.g., caused by viral infection, pathogenic infection, cancer, etc.), autoimmune diseases, grafts, diabetes, inflammatory diseases, cancers, allergies, asthma, psoriasis, urticaria, eczema and the like.

In a particular embodiment, the invention is directed to methods for stimulating an immune response in a subject in need thereof, comprising administering a cofactor inhibitor or immunogen to said subject.

In another particular embodiment, the invention is directed to methods for treating an immunodeficiency or an associated disorder in a subject in need thereof, comprising administering a cofactor inhibitor or immunogen to said subject, preferably in an amount effective to maintain/restore resistance of CD4 T cells to inactivation by PLA2-GIB. Immunodeficiencies and associated disorders designate any condition or pathology characterized by and/or caused by a reduced immune function or response in a subject. Immunodeficiencies may be caused by e.g., viral infection (e.g., HIV, hepatitis B, hepatitis C, etc.), bacterial infection, cancer, or other pathological conditions. The term "immunodeficiency-associated disorder" therefore designates any disease caused by or associated with an immunodeficiency. The invention is particularly suitable for treating immunodeficiencies related to CD4-T cells, and associated diseases.

As used herein, "treatment" or "treat" refers to clinical intervention in an attempt to alter the natural course of the individual being treated, and can be performed either for preventive or curative purpose. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, compositions and methods of the invention are used to delay development of a disease or disorder or to slow the progression of a disease or disorder.

The duration, dosages and frequency of administering compounds or compositions of the invention may be adapted according to the subject and disease. The treatment may be used alone or in combination with other active ingredients, either simultaneously or separately or sequentially.

The compounds or compositions according to the invention may be administered in various ways or routes such as, without limitation, by systemic injection, intramuscular, intravenous, intraperitoneal, cutaneous, subcutaneous, dermic, transdermic, intrathecal, ocular (for example corneal) or rectal way, or by a topic administration on an inflammation site, and preferably by intramuscular or intravenous injection.

A typical regimen comprises a single or repeated administration of an effective amount of a cofactor or modulator over a period of one or several days, up to one year, and including between one week and about six months. It is understood that the dosage of a pharmaceutical compound or composition of the invention administered *in vivo* will be dependent upon the age, health, sex, and weight of the recipient (subject), kind of concurrent treatment, if any, frequency of treatment, and the nature of the pharmaceutical effect desired. The ranges of effectives doses provided herein are not intended to be limiting and represent preferred dose ranges. However, the most preferred dosage will be tailored to the individual subject, as is understood and determinable by one skilled in the relevant arts (see, e.g., Berkowet et al., eds., The Merck Manual, 16th edition, Merck and Co., Rahway, N.J., 1992; Goodmanetna., eds., Goodman and Cilman's The pharmacological Basis of Therapeutics, 10th edition, Pergamon Press, Inc., Elmsford, N.Y., (2001)).

### Viral diseases

The invention may be used to treat viral diseases or viral infection in mammals.

Examples of viruses that can be treated with the methods provided herein include, but are not limited to, enveloped viruses such as members of the following viral families: Retroviridae (e.g., HIV (such as HIV1 and HIV2), MLV, SIV, FIV, Human T-cell leukemia viruses 1 and 2, XMRV, and Coltiviruses (such as CTFV or Banna virus)); Togaviridae (for example, alphaviruses (such as Ross River virus, Sindbis virus, Semliki Forest Virus, O'nyong'nyong virus, Chikungunya virus, Eastern equine encephalitis virus, Western equine encephalitis virus, Venezuelan equine encephalitis virus) or rubella viruses); Flaviridae (for example, dengue viruses, encephalitis viruses (such as West Nile virus or Japanese encephalitis virus), yellow fever viruses); Coronaviridae (for example, coronaviruses such as SARS virus or Toroviruses); Rhabdoviridae (for example, vesicular stomatitis viruses, rabies viruses); Paramyxoviridae (for example, parainfluenza viruses, mumps virus, measles virus, respiratory syncytial virus, sendai virus, and metopneumovirus); Orthomyxoviridae (for example, influenza viruses); Bunyaviridae (for example, Hantaan virus, bunya viruses (such as La Crosse virus), phleboviruses, and Nairo viruses); Hepadnaviridae (Hepatitis B viruses); Herpesviridae (herpes simplex virus (HSV) 1 and HSV-2, varicella zoster virus, cytomegalovirus (CMV), HHV-8, HHV-6, HHV-7, and pseudorabies virus); Filoviridae (filoviruses including Ebola virus and Marburg virus) and Poxyiridae (variola viruses, vaccinia viruses, pox viruses (such as small pox, monkey pox, and Molluscum contagiosum virus), yatabox virus (such as Tanapox and Yabapox)). Non-enveloped viruses can also be treated with the methods provided herein, such as members of the following families: Calciviridae (such as strains that cause gastroenteritis); Arenaviridae (hemorrhagic fever viruses such as LCMV, Lassa, Junin, Machupo and Guanarito viruses); Reoviridae (for instance, reoviruses, orbiviruses and rotaviruses); Birnaviridae; Parvoviridae (parvoviruses, such as Human bocavirus adeno-associated virus); Papillomaviridae (such as papillomaviruses); Papovaviridae (papilloma viruses, polyoma viruses); Adenoviridae (adenoviruses); Picornaviridae (enteroviruses, enteric viruses, Poliovirus, coxsackieviruses, hepatoviruses, cardioviruses, aptoviruses, echoviruses, hepatitis A virus, Foot and mouth disease virus, and rhinovirus) and Iridoviridae (such as African swine fever virus). Other viruses that can be treated using the methods provided herein include unclassified viruses (for example, the etiological agents of Spongiform encephalopathies, the agent of delta hepatitis (thought to be a defective satellite of hepatitis B virus), the agents of non-A, non-B hepatitis (for instance, Hepatitis C); calciviruses (such as Norovirus, Norwalk and related viruses); Hepeviruses (such as Hepatitis E, JC and BK viruses) and astroviruses).

In a particular embodiment, the invention relates to methods of treating HIV infection in a subject by administering a cofactor inhibitor to the subject, optionally in combination with a sPLA2-GIB inhibitor. In some embodiments the subject is an early HIV patient and the methods results in increasing the probability that the patient is a HIV controller. In some embodiments the subject is a patient with low immunoreconstitution after antiretroviral treatment and/or with severe idiopatic CD4 T lymphopenia (ICL). The invention also relates to a method for increasing CD4-T cell activity in a HIV-infected subject by inhibiting sPLA2-GIB and a cofactor in the subject, preferably by administering a sPLA2-GIB inhibitor and a cofactor inhibitor to the subject.

For treating HIV infection, preferred cofactor inhibitors are selected from (i) antibodies or variants thereof or nucleic acids that bind gClqR or a polypeptide of SEQ ID NO: 3, and (ii) peptides comprising a fragment of SEQ ID NO: 3.

In another particular embodiment, the invention relates to methods of treating HCV infection in a subject by administering a cofactor inhibitor to the subject, optionally in combination with a sPLA2-GIB inhibitor.

For treating HCV infection, preferred inhibitors are selected from (i) antibodies or variants thereof or nucleic acids that bind gClqR or a polypeptide of SEQ ID NO: 43, and (ii) peptides comprising a fragment of SEQ ID NO: 43.

### Bacterial / fungal diseases

Examples of infectious bacteria that can be treated with the methods provided herein include, but are not limited to, any type of Gram-positive (such as *Streptococcus, Staphylococcus, Corynebacterium, Listeria, Bacillus* and *Clostridium*) or Gram-negative bacteria (such as *Porphyromonas, Salmonella, Shigella, Enterobacteriaceae, Pseudomonas, Moraxella, Helicobacter, Stenotrophomonas, acetic acid bacteria, alpha-proteobacteria, Escherichia coli, Neisseria gonorrhoeae, Neisseria meningitidis, Moraxella catarrhalis, Hemophilus influenzae, Klebsiella pneumoniae, Legionella pneumophila, Pseudomonas aeruginosa, Proteus mirabilis, Enterobacter cloacae* and *Serratia marcescens.* Exemplary infectious bacteria include, but are not limited to: *Porphyromonas gingivalis, Porphyromonas somerae, Terrisporobacter glycolicus, Aggregatibacter actinomycetemcomitans, Aggregatibacter aphrophilus, Bacteroides fragilis, Helicobacter pyloris, Borelia burgdorferi, Legionella pneumophilia, Mycobacteria* sps (such as *M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes* (Group A *Streptococcus*), *Streptococcus agalactiae* (Group B *Streptococcus*), *Streptococcus* (viridans group), *Streptococcus faecalis, Streptococcus bovis, Streptococcus* (*anaerobic* sps.), *Streptococcus pneumoniae,* pathogenic *Campylobacter* sp., *Enterococcus* sp., *Haemophilus influenzae, Bacillus anthracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira,* and *Actinomyces israelli.*

Examples of infectious fungi that can be treated with the methods provided herein include, but are not limited to, *Cryptococcus neoformans, Histoplasma capsulatum, Coccidioides immitis, Blastomyces dermatitidis, Chlamydia trachomatis, Candida albicans and Candida glabrata.*

In a particular embodiment, the invention relates to methods of treating Staphylococcus infection in a subject, particularly S. aureus infection, by administering a cofactor inhibitor or immunogen to the subject, optionally in combination with a sPLA2-GIB inhibitor.

For treating Staphylococcus infection in a subject, particularly S. aureus infection, preferred inhibitors are selected from (i) antibodies or variants thereof or nucleic acids that bind gClqR or a polypeptide of SEQ ID NO: 44, and (ii) peptides comprising a fragment of SEQ ID NO: 44.

In a particular embodiment, the invention relates to methods of treating Listeria infection in a subject, particularly L. monocytogenes infection, by administering a gClqR inhibitor to the subject, optionally in combination with a sPLA2-GIB inhibitor.

For treating Listeria infection in a subject, particularly L. monocytogenes infection, preferred gC1qR inhibitors are selected from (i) antibodies or variants thereof or nucleic acids that bind gClqR or a polypeptide of ID NO: 52, and (ii) peptides comprising a fragment of ID NO: 52.

In a particular embodiment, the invention relates to methods of treating Streptococcus infection in a subject, particularly S. pneumoniae infection, by administering a cofactor inhibitor to the subject, optionally in combination with a sPLA2-GIB inhibitor.

For treating Streptococcus infection in a subject, particularly S. pneumoniae infection, preferred inhibitors are selected from (i) antibodies or variants thereof or nucleic acids that bind gClqR or a polypeptide of ID NO: 53, and (ii) peptides comprising a fragment of ID NO: 53.

In a particular embodiment, the invention relates to methods of treating Bacillus infection in a subject, particularly B. cereus infection, by administering a gClqR inhibitor to the subject, optionally in combination with a sPLA2-GIB inhibitor.

For treating Bacillus infection in a subject, particularly B. cereus infection, preferred gClqR inhibitors are selected from (i) antibodies or variants thereof or nucleic acids that bind gClqR or a polypeptide of ID NO: 54, and (ii) peptides comprising a fragment of ID NO: 54.

### Parasitic diseases

Examples of infectious parasites that can be treated with the methods provided herein include, but are not limited to *Plasmodium falciparum* and *Toxoplasma gondii.*

In a particular embodiment, the invention relates to methods of treating *Plasmodium falciparum* infection in a subject by administering a cofactor inhibitor to the subject, optionally in combination with a sPLA2-GIB inhibitor.

For treating *Plasmodium falciparum* infection in a subject, preferred inhibitors are selected from (i) antibodies or variants thereof or nucleic acids that bind gClqR or a polypeptide of SEQ ID NO: 55, and (ii) peptides comprising a fragment of ID NO: 55.

### Other disorders

The invention may be used to improve the immune system in any mammal in need thereof. It is suitable to correct undesirable effects, such as iatrogene effect of drugs, toxins, pollutants, pesticides, etc.

The invention may be used in any mammal, particularly any human.

Further aspects and advantages of the invention will be disclosed in the following experimental section.

### Examples

### MATERIALS AND METHODS

*Recombinant proteins and peptides-* Human PLA2-GIB was produced in E. coli (gift Gerard Lambeau, purity >98%) or in CHO-S (purity >98%). HIV-1 gp41 MN recombinant protein was obtained from Antibodies onlines (gp41 MN (565-771Delta642-725), ABIN2129703, lot 93-482, purity >95%), and PEP3 peptide NH2-PWNASWSNKSLDDIW-COOH and control peptide (CTL) NH2-PWNATWTQRTLDDIW-COOH were ordered from Covalab (purity >98%). Complement component C1q from human serum was obtained from Sigma (C1740, purity >95%). HCV core protein was obtained from Prospec (HCV-011, purity >95%) in PBS buffer with 0.002%SDS and the specificity of effect due to HCV core protein was evaluated by comparison with similar dilution of PBS SDS 0.002%). Staphylococcus aureus protein A was obtained from Sigma (P6031).

*gp41 immunodepletion of viremic patient and healthy donor plasma-* 1ml of viremic patient plasma or healthy donor plasma were incubated with 100µg of goat anti-gp41 polyclonal antibody (PA21719, Fisher) or the control goat polyclonal antibody (preimmune, AB1 08-C, R&D) in 1.5ml Eppendorf tubes overnight on a rotor at 4°C. Then 200µl of Protein G sepharose 4 Fast Flow beads (17-0618-01, GE healthcare), washed three times in PBS BSA 1%, were added each sample for 3h on a rotor at 4°C. To remove beads, samples were first centrifugated at 400 x g for 2 min at 4°C, the supernatant was collected and then centrifuged at 16,100 x g for 15 min at 4°C. As the control goat polyclonal antibody initially contained sodium azide it was washed with 5 times with PBS on 10kDa Amicon to remove sodium azide before proceeding to immunodepletion.

*AT-2 inactivated HIV-1 particles-* To preserve the conformational and functional integrity of HIV particles, inactivation was done with 2,2-dithiodipyridine (AT-2; 43791, Sigma) on HIV-1 NDK (T-tropic) particles and prepared on PHA-stimulated PBMCs as described in (Rossio et al., J Virol. 1998). 2,2-dithiodipyridine (aldrithiol-2; AT-2) covalently modify the essential zinc fingers in the nucleocapsid (NC) protein of human immunodeficiency virus type 1 (HIV-1). HIV-1 particles were inactivated twice with 300µM of AT-2 for 1h at 37°C in a water bath followed by 2h on ice.
In parallel the supernatant of PHA-stimulated PBMCs was treated as HIV-1 NDK-infected cells supernatant to serve as Mock control (without HIV-1 particles). Inactivation of HIV particles was confirmed by an undetectable TCID50 in the infectivity assay. HIV particle concentration was determined by anti-HIV-1 gag p24 ELISA assay (HIV-1 Gag p24 Quantikine ELISA Kit, DHP240, R&D systems biotechne). HIV-1 particles were used at 5000, 500, 50 and 5 pg of p24/10e⁶ cells. 5000µg of p24/10e⁶ cells (1754 pg of p24/3.5x10e⁵ cells) that is equivalent to 1 particle by cells (multiplicity of infection, MOI, of 1).

*Purification of Human CD4 T-lymphocytes-* Venous blood was obtained from healthy volunteers through the EFS (Etablissement Français du Sang, Centre Necker-Cabanel, Paris). CD4 T-cells were purified from whole blood using RosetteSep Human CD4+ T cell Enrichment Cocktail (Stem Cell, 15062). This cocktail contains mouse and rat monoclonal antibodies purified from mouse ascites fluid or hybridoma culture supernatant, by affinity chromatography using protein A or Protein G sepharose. These antibodies are bound in bispecific tetrameric antibody complexes which are directed against cell surface antigens on human hematopoietic cells (CD8, CD16, CD19, CD36, CD56 CD66b, TCRγ/δ) and glycophorin A on red blood cells. The rosetteSep antibody cocktail crosslinks unwanted cells in human whole blood to multiple red blood cells, forming immunorosettes. This increases the density of unwanted cells, such that they pellet along with the free red blood cells when centrifuged over a buoyant density medium such as lymphocytes separation medium (Eurobio, CMSMSL01-01).

Whole blood was incubated with RosetteSep Human CD4+ T cell Enrichment Cocktail at 50µl/ml for 20 minutes at room temperature under gentle shaking (100 rpm), diluted with an equal volume of PBS+2% foetal bovine serum (FBS) and mixed gently. The diluted samples were centrifuged 20 minutes at 1200 X g on top of lymphocytes separation medium. The enriched cells were then collected from the density medium at plasma interface and washed twice with PBS+2% FBS. Cells were subsequently resuspended in RPMI 1640 medium (Lonza) supplemented with 5% FBS, 50 mM HEPES pH 7.4, glutamine, penicillin, streptomycin and fungizone (complete medium), counted with a Moxi Z mini automated cell counter (ORFLO, MXZ000). Cells suspension was adjusted at 7x10⁶ cells/ml and equilibrated at least 2 h at 37°C in a 5% CO2 humidified atmosphere.

The enriched CD4-T cell population was controlled by flow cytometry on a cytoflex (Beckman coulter). The quiescence of recovered CD4 T-cells was controlled by the low level of IL-2Rα (CD25). CD4 T cells were labeled with anti-Human CD3 eFluor780 (eBioscience, clone UCHT1, 47-0038-42), anti-Human CD25-PE (Biolegend, clone BC96, 302605) and anti-human CD4-PerCP (BD, clone SK3, 345770). The enriched CD4-T cell population contains >95% CD3+CD4+ and less than 8% of CD25+.

*PLA2-GIB bioassay on CD4 T cells and labelling of specific proteins for optical microscopy-* Equilibrated purified CD4 T-cells were loaded (3.5x10⁵cells/50µl in complete medium) on poly-L-Lysine-coated (Sigma, P8920) round coverslips (14mm-diameter, Marienfeld) in 24-well polystyrene plates at 37°C in a thermo-regulated water and mixed with 50µl of a suspension in PBS BSA1% containing peptides, recombinant proteins together with recombinant PLA2-GIB or not or containing viremic patient plasma (1 or 3%) or healthy donor plasma. The cells suspension was either pretreated with 40µl of peptides, recombinant protein or HIV-1 NDK particles or mock dilutions in PBS BSA1% for 15 minutes with subsequent addition of 10µl PLA2-GIB (5nM at the end) for 30 minutes or directly treated with 50µl of dilution in PBS BSA 1% with peptides or recombinant protein together with PLA2-GIB (5nM at the end) for 45 minutes. Cells were activated for 15 minutes with 2 nM recombinant glycosylated human IL-7 (Accrobio System). Cells supernatant was removed and cells were fixed by addition of 500µl of a 4% paraformaldehyde solution in PBS (Fisher, PFA 32% Electron Microscopy Science, 15714) for 15 minutes at 37 °C and then permeabilized for 20 min in 500µl of ice-cold 90% methanol/water solution.

Cells were then rehydrated for 15 min in PBS plus 5% fetal bovine serum (FBS) and then labeled. Thus, slides were washed twice after methanol treatment in PBS and rehydrated for 15 min in PBS supplemented with 5% FBS at room temperature. Slides were labelled with primary antibodies (1/120) in 60 µl of PBS 5% FBS for 1h, washed in PBS buffer 15 times, 5 times in PBS/FBS buffer and then stained with secondary antibodies (1/300) for 1h. Slides were washed 5 times in PBS 5% FBS buffer, rinsed 15 times in PBS and then mounted in fresh Prolong Gold Antifade (ThermoFisher Scientific, P36930) mounting medium for confocal microscopy. The primary antibodies used consisted of rabbit anti-pSTAT5 (pY694, 9359, Cell Signalling), mouse anti-CD4 (BD Pharmingen, 555344) and secondary antibodies were Donkey anti-mouse IgG-AF488 (Invitrogen, A21202) and Donkey anti-rabbit IgG-AF555 (Invitrogen, A31572). *Blocking of gC1qR with anti-gC1qR antibodies 60.11 and 74.5.2-* Equilibrated purified CD4 T-cells were preincubated for 30 min with anti-gClqR 60.11 (epitope 75-96, Santa Cruz, sc-23884), 74.5.2 (epitope 204-218, Abcam, ab125132) (Ghebrehiwet B et al., Adv Exp Med Biol. 2013) or control IgG1 (mouse IgG1 control Isotype, eBioscience/Affymetrix, 16-4714) and loaded (3.5x10⁵ cells/60µl in complete medium) on poly-L-Lysine-coated (Sigma, P8920) round coverslips (14mm-diameter, Marienfeld) in 24-well polystyrene plates at 37°C in a thermo-regulated water. Cells were further treated for 45 min with C1q (Sigma C1740, purity >95%, 10µg/ml), PEP3 peptide (0.5µg/ml) with or without PLA2-GIB at 5nM or viremic patient plasma 1% or 3% in final volume of 100µl. Then cells were stimulated with IL-7 and treated as decribed above to analyze pSTAT5 NT by confocal microscopy.

*Confocal Microscopy-* Images were acquired above the diffraction limit on an inverted laser scanning confocal microscope (LSM700, Zeiss), with an oil-immersion plan-apochromatic 63x/1.4 NA objective lens (Zeiss) for PFA-fixed cells. Images were acquired and analyzed with the ZEN software (Zeiss).

*PLA2-GIB enzymatic assay on [3H] arachidonic acid labelled CD4 T cells-* Purified CD4 T-cells were incubated for 16h at 2x10⁶ cells/ml with 1µCi/ml of arachidonic acid [5,6,8,9,11,14,15-³H(N)] (Perkin Elmer, NET298Z250UC) in RPMI 1640 medium (Lonza) supplemented with 10% FBS, 50 mM HEPES pH 7.4, glutamine, penicillin, streptomycin and fungizone at 2 ml/well in 6-well plates at 37°C in a 5% CO2 humidified atmosphere. Cells were washed twice with RPMI with 10% FBS by centrifugation at 580xg for 10 minutes at room temperature and then frozen in 90% FBS 10% DMSO at 10⁷ cells/ml/vial at -80°C. Percent of [3H] arachidonic acid in CD4 T cells is the (1 minus ratio of [3H] arachidonic acid in the supernatant of CD4 T cells without cells (cpm/ml) on total [3H] arachidonic acid in supernatant and cells (cpm/ml).

To test PLA2-GIB activity on [3H] arachidonic acid labelled CD4 T lymphocytes, cells were unfrozen in 10% FBS RPMI preheated at 37°C by centrifugation at 580xg for 10 minutes at room temperature, washed twice in 2.5% FBS RPMI, and equilibrated at 2x10⁵ CD4 T cells/400µl/well in 24-well polystyrene plates for 1h30 at 37°C in a 5% CO2 humidified atmosphere. Then 100µl of recombinant proteins (gp41 MN (565-771Delta642-725), Antibodies online, ABIN2129703; HCV core protein, HCV-011, Prospec) or vehicle dilution in 2.5% FBS RPMI was added to each well for 2h. Cells and supernatant were collected in eppendorf tubes and centrifuged at 580xg for 10 minutes at room temperature. The [3H] arachidonic acid released in cell supernatant was quantified in 300µl with 16 ml of Ultima gold (Perkin Elmer, 6013329) in low diffusion vials (Perkin Elmer, 6000477) on a counter (tri-Carb 2800 TR liquid scintillation analyzer, Perkin Elmer).

### RESULTS AND DISCUSSION

### 1.Viremic patient plasma increases the activity of PLA2-GIB on CD4 T cells

We have shown previously that treatment of CD4 T cells with 75nM of PLA2G1B alone significantly decreases the nuclear translocation of phosphoSTAT5 (pSTAT5 NT) induced by IL-7 while treatment with 5nM of PLA2G1B does not affect this response to IL-7 (Buffer, Figure 1A). The endogenous PLA2G1B-depleted viremic plasma does not affect phosphosSTAT5 translocation in response to IL-7. Notably addition of 5nM of PLA2G1B in 1% of endogenous PLA2G1B-depleted viremic plasma results in 40% of inhibition pSTAT5 NT while healthy donor plasma similarly treated has no effect (n=4 independent donors, *p*<0.0001, Figure 1A). These results demonstrate that viremic plasma contains a cofactor that sensitizes CD4 T cells to inhibition by PLA2G1B.

To identify the molecular weight of this viremic plasma cofactor we fractionated viremic plasma on filter with 30kDa and 10kDa cut-off. As shown on figure 1B, the fraction of endogenous PLA2G1B-depleted viremic plasma which contains products of more than 10kDa and less than 30kDa increases PLA2G1B activity on CD4 T cells but not the same fraction from healthy donor plasma. The fraction of endogenous PLA2G1B-depleted viremic plasma which contains products of more than 30kDa and the fraction which contains products of less than 10kDa have no effect on PLA2G1B activity. Thus, viremic plasma patient contains a cofactor with a molecular weight between 10kDa and 30kDa that sensitizes CD4 T cells to inhibition by PLA2G1B under experimental conditions where PLA2G1B concentration alone is not sufficient to affect pSTAT5 NT in response to IL-7.

### 2.HIV-1 inactivated viral particles sensitize CD4 T cells to PLA2-GIB inhibitory activity on response to IL-7

To test the hypothesis that HIV-1 viral products could play a role in the cofactor activity of viremic plasma we first investigated the effect of HIV-1 particles on pSTAT5 NT response to IL-7 in healthy donor CD4 T cells. We used HIV-1 particle of a T-tropic HIV-1 NDK virus previously inactivated with AT-2 to test the effect of viral proteins on CD4 T cells in absence of infection. To test the cofactor activity, CD4 T cells were exposed to different amount of HIV particles (MOI 1, 0.1, 0.01 and 0.001) alone or in presence to an amount of PLA2-GIB (5nM) that does not inhibit phosphoSTAT5 nuclear translocation in response to IL-7 (Figure 2). pSTAT5 NT in response to IL-7 was more than 92% without PLA2-GIB, biologically similar with 5nM of PLA2-GIB and only at 50% and 10% with 75nM and 250nM of PLA2-GIB as expected. HIV-1 particles alone do not affect pSTAT5 NT in response to IL-7. Of note HIV-1 particles results in a dose-response inhibition of pSTAT5 NT in presence of 5nM of PLA2-GIB (48% of pSTAT5 NT with 5pg/ml of p24 (MOI=0.001) to only 8% of pSTAT5 NT with an 5000pg/ml of p24 (MOI =1), *p*<0.001, Figure 2) while similar dilutions of control (Mock) with 5nM of PLA2-GIB have no effect on pSTAT5 NT in response to IL-7. These results demonstrate that some viral components could play the role of cofactor that sensitize CD4 T cells to PLA2-GIB activity as observed in viremic patient plasma.

### 3.HIV-1 gp41 protein increases PLA2-GIB inhibitory activity on pSTAT5 NT in CD4 T cells stimulated with IL-7

We analyzed pSTAT5 NT response to IL-7 in cells pretreated with a dose of PLA2-GIB that cannot inhibit pSTAT5 NT in absence of cofactor (5nM), together or not with a recombinant gp41 protein or with gp41 protein alone, without PLA2-GIB (w/o GIB). As shown on Figure 3, gp41 protein alone as a minor inhibitory effect on pSTAT5 NT response to IL-7 at 0.5µg/ml of gp41 with only 10% of inhibition and less than 8% of inhibition with 0.25 to 0.05 µg/ml of gp41 (Figures 3A and 3B). By a striking contrast, in presence of 5nM of PLA2-GIB, 0.5µg/ml of gp41 protein resulted in more than 60% of inhibition of pSTAT5 NT (Figure 3B) with a dose-dependent inhibition to 18% of inhibition with 0.005µg/ml of gp41 (Figure 3A).

### 4.HIV-1 gp41 protein plays a critical role in the inhibitory activity of viremic patient plasma on pSTAT5 NT in CD4 T cells stimulated with IL-7

To verify that gp41 protein could be a cofactor of PLA2-GIB in viremic patient plasma, we depleted viremic patient plasma with polyclonal antibody against gp41 (pAb anti-gp41) or control polyclonal antibody (pAb ctrl). Healthy donor plasma was similarly treated as negative control. As presented on Figure 4, the inhibition of pSTAT5 NT was 49% with 75nM and 79% with 250nM of PLA2-GIB as expected and 39% with 1% and 54% with 3% of viremic patient plasma without antibody. Healthy donor plasma had no inhibitory effect on pSTAT5 NT in response to IL-7 without antibody, with control polyclonal antibody or anti-gp41 polyclonal antibody which demonstrates that antibodies have no toxicity on CD4 T cells (Figure 4). Treatment of viremic patient plasma with control polyclonal antibody does not change the inhibitory activity with 43% and 55 % of inhibition with 1% and 3% of plasma respectively (Figure 4). Notably, immunodepletion with anti-gp41 polyclonal antibody almost abrogated the inhibitory activity of viremic patient plasma with 6% and 10% of residual inhibitory activity with 1% and 3% of immunodepleted plasma (p<0.001 pAb anti-gp41 vs pAb ctrl treated plasma, Figure 4). Altogether these results demonstrate that gp41 is a cofactor of PLA2-GIB in viremic patient plasma.

### 5.The PEP3 motif in gp41 inhibits pSTAT5 NT in CD4 T cells stimulated with IL-7

CD4 T cells were exposed to a 15 aminoacids peptide domain of gp41 containing a potential gClqR binding element. The peptide contains SWSNKS motif. The cells were also exposed to a control (CTL) peptide (Figure 5A), together with 5nM of PLA2-GIB (5nM GIB) or not (w/o). While CTL peptide alone or with PLA2-GIB or PEP3 alone have no effect on pSTAT5 NT, treatments with PEP3 and 5nM of PLA2-GIB resulted in a PEP3 dose-dependent inhibition of pSTAT5 NT from 51% to 18% of inhibition with 2.5 µg/ml to 0.025 µg/ml of PEP3 respectively (Figure 5B). As summarized on Figure 5C, treatment with 0.5µg/ml of PEP3 alone only resulted in 5% of inhibition of pSTAT5 NT in CD4 T cells while treatment with 0.5µg/ml of PEP3 together with 5nM PLA2-GIB resulted in 55% of inhibition (p<0.05, n=3 donors).

### 5.The cofactor activity of PEP3 on PLA2G1B and the inhibitory activity of viremic patient plasma are dependent on gClqR

We hypothesized that gClqR could play a role in the inhibitory activity of viremic patient plasma. To study the role of gClqR in PLA2-GIB inhibition of pSTAT5 NT, we tested the effect of Clq, the natural ligand of gClqR, on PLA2-GIB activity. We found that C1q alone was able to inhibit 40% pSTAT5 NT (*p*<0.001). PLA2-GIB addition to C1q increases this inhibitory activity to 75-85% of inhibition, (p<0.01, Figure 6A) and C1q effect as well as cofactor effect on PLA2-GIB was significantly inhibited with two different anti-gClqR antibodies that restore 75% of response (60.11 and 75.4.2 anti-gClqR antibodies vs IgGlctrl with C1q and 5nM PLA2-GIB, *p*<0.001, Figure 6A). Notably the anti-gClqR antibody 74.5.2 restore 54% of pSTAT5 NT in presence of PEP3 and PLA2-GIB (Figure 6B, *p*<0.0001) and 32% of pSTAT5 NT in cells treated with 1% of viremic patient plasma (Figure 6C, *p*<0.0001). By contrast, the control antibody (IgGl ctrl) does not inhibit PEP3 cofactor activity on PLA2-GIB nor viremic patient plasma effect (Figures 6B and 6C).

### 6.gp41 protein sensitizes CD4 T cells membranes to PLA2G1B enzymatic activity

To study PLA2-GIB effect on CD4 T cells membranes we developed a new enzymatic assay in which CD4 T cells are labelled with [3H] arachidonic acid. When these cells are exposed to PLA2-GIB the enzymatic activity on CD4 T cells releases [3H] arachidonic acid. The quantification of [3H] arachidonic acid allowed us to quantify PLA2-GIB activity.

As we observed above that gp41 protein can increase PLA2-GIB inhibitory activity on pSTAT5 NT, we postulated that gp41 could increase PLA2-GIB enzymatic activity on CD4 T cells membranes. Indeed, PLA2-GIB enzymatic activity is highly and significantly increased when gp41 is present and in a gp41 dose-dependent manner (*p*<0.01 and *p*<0.001, Figure 7). Gp41 treatment alone has no effect on [3H] arachidonic acid release by CD4 T cells. Treatments with 0.5 to 5µg/ml of gp41 resulted in a 2.2 to 21-fold increase of 63nM of PLA2-GIB activity and 1.5 to 11.6-fold increase of 200nM of PLA2-GIB activity on [3H] arachidonic acid release by CD4 T cells with a maximum at 5µg/ml of gp41. Treatment with 5µg/ml of gp41 can increase the activity of PLA2-GIB more than 70-Fold on some donor.

### 7.Other PLA2-GIB cofactors

Our demonstration that gClqR is a sensor of PLA2-GIB cofactor led us to investigate other gClqR ligands.

Table 2 lists 30 different molecules that bind to gClqR and can thus affect PLA2-GIB activity. About half of these molecules are derived from pathogens: 9 are viral proteins, 4 are bacterial components and one is the Plasmodium falciparum parasite (Table 2). One molecule, LyP-1, is an artificial gClqR ligand and the other 15 are endogenous components, five from serum and 10 from cells. Altogether these results suggest that PLA2-GIB activity can be modulated by various distinct pathogen components and endogenous factors, and that this pathway is a general mechanism of pathogenesis.

### 8. HCV core protein sensitizes CD4 T cells membranes to PLA2-GIB enzymatic activity

We analyzed PLA2G1B enzymatic activity on CD4 T cells in the presence of recombinant HCV core protein (Figure 8). HCV core protein contains a gClqR binding element (see table 2). Our results show that HCV core protein alone slightly induces the release of [3H] arachidonic acids by CD4 T cells at 10 and 20µg/ml (Figure 8A). Interestingly treatments of CD4 T cells with PLA2G1B and HCV core protein highly increases PLA2G1B enzymatic activity with a 26-fold and 36-fold increase of activity of 63nM of PLA2G1B and 16-fold and 26-fold increase of activity of 200nM of PLA2G1B at 10 and 20µg/ml of HCV core protein (Figure 8A). As summarized on Figure 8B, treatment with 10µg/ml of HCV core protein alone slightly and significantly increases the release of [3H] arachidonic acids by CD4 T cells. Furthermore, HCV core protein is a very potent PLA2G1B cofactor with 26-fold and 16-fold increase activity of 63nM and 200nM of PLA2G1B respectively (p<0.001, n=3 donors). These results show that HCV core protein can sensitize CD4 T cells to PLA2G1B inhibition, thus leading to an inhibition of CD4 T cells function in patients with hepatitis C infection.

### 9. Staphylococcus aureus protein A (SA protein A) sensitizes CD4 T cells to PLA2G1B enzymatic activity

We analyzed the effect of the SA protein A, another gClqR binding protein (Table 2), on PLA2G1B enzymatic activity on CD4 T cells (Figure 9). As observed with HCV core protein, SA protein A alone slightly induces the release of [3H] arachidonic acids by CD4 T cells at 10, 25 and 50µg/ml (Figure 9A, p<0.01). Notably treatments of CD4 T cells with PLA2G1B and SA protein A significantly increases PLA2G1B enzymatic activity 1.5-fold to 3-fold more activity of 200nM of PLA2G1B at 10 to 50µg/ml of SA protein A (Figure 9B, p<0.0001). These results show that SA protein A can sensitize CD4 T cells to PLA2G1B inhibition, thus leading to an inhibition of CD4 T cells function in patients with Staphylococcus aureus infection. These results also complete the above observation with the viral protein HCV core and demonstrate that bacteria proteins that binds to gClqR could be PLA2G1B cofactors. Altogether HCV core protein and SA protein A experiments suggest that gClqR activation/PLA2G1B sensitization could be a general mechanism by which pathogens act.

### 10. Identification of gClqR-binding domain-containing proteins that can act as PLA2-GIB cofactors

We screened protein database with PEP3 peptide sequence to identify other proteins containing a gClqR-binding element. 42 Proteins from 27 different bacteria species and one fungus (Candida glabrata) were identified, one was from ananas, another from Caenorhabditis elegans and the last one was from human (Table 1). Among them, we identified 11 proteins from 9 human pathogens (8 bacteria and 1 fungus) that could regulate PLA2G1B activity, as summarized in Table 3. These pathogens have been associated with cancer, autoimmune and neurodegenerative diseases. For instance, Porphyromonas gingivalis infection is associated with pancreatic cancer, Rheumatoid arthritis, Alzheimer's disease and Candida glabrata infection is associated with cutaneous candidiasis in HIV/AIDS patients, patients with cancer and chemotherapy treatment and organ transplantation.

**Table 1**

| **PROTEIN NAME** | **ACCESSION NUMBER** | **SPECIES** | **SEQUENCE OF gC1qR BINDING ELEMENT** | **SEQ ID NO:** |
|---|---|---|---|---|
| gp41 | AAC31817.1 | HIV | PWNASWSNKSLDDIW | 3 (residues 97-111) |
| UNKNOWN | WP_077094164.1 | Porphyromonas gingivalis | AWNAIWINRKYEQID | 4 |
| UDP-glucose 4-epimerase | SJM20449.1 | Porphyromonas gingivalis | GIAESWPNSLDDSCA | 5 |
| L-threonine 3-dehydrogenase | WP_013815975.1 | Porphyromonas gingivalis | GIAESWPNSLDDSCA | 6 |
| TonB-dependent receptor | MP_097552718.1 | Porphyromonas gingivalis | SFLKSWFNNSLVDIG | 7 |
| UNKNOWN | WP_097552551.1 | Porphyromonas gingivalis | SGEGGWSNGSLVDIM | 8 |
| DNA polymerase III subunit gamma/tau | SJM20595.1 | Porphyromonas gingivalis | YELDAASNNSVDDIR | 9 |
| Multidrug transporter AcrB | WP_097555277.1 | Porphyromonas gingivalis | AALGKTLVKSLDDIP | 10 |
| Peptide ABC transporter permease | AIF49259.1 | Dyella j aponica | PWNASWSDKFYENSL | 11 |
| Peptide ABC transporter substrate binding protein | WP_019421834.1 | Paenibacillus sp. | AIHASWSNTSYEVID | 12 |
| Peptide ABC transporter substrate binding protein | OJX83063.1 | Mesorhizobium sp. | PWNAGWSNARFDELC | 13 |
| MC73_05565 | KGY43685.1 | Proteus mirabilis | PWNAIWSAKNTTVDS | 14 |
| Chitinase | WP_068978097.1 | Aeromonas sp. | PWNASWSAAGVGAHA | 15 |
| TonB-dependent receptor | KZY48959.1 | Pseudoalteromonas sp. | WFNASWKDKSYSTVW | 16 |
| TonB-dependent receptor | WP_036212264.1 | Lysobacter arseniciresistens | PWNASWSVRHISELE | 17 |
| LVIVD repeat protein | EMY15726.1 | Leptospira weilii str. | PWNASWSYVLDSAWS | 18 |
| AMK72_12855 | KPK43807.1 | Planctomycetes bacterium | PWNGSWSNDAWGPGT | 19 |
| Peptide ABC transporter substrate-binding protein | OJX83063.1 | Mesorhizobium sp. | PWNAGWSNARFDELC | 20 |
| UNKNOWN | WP_030088081.1 | Streptomyces baarnensis | PWNAGWSLKSSGKSA | 21 |
| HMPREF0183_2345 | EFG46376.1 | Brevibacterium mcbrellneri | PWNAWWSNRSMIADV | 22 |
| UNKNOWN | WP_048669463 | Vibrio crassostreae | AWNESWSNKSFHNGA | 23 |
| UNKNOWN | WP_070707834.1 | Porphyromonas sp. HMSC077F02 | EFNANWSNKFYLYNQ | 24 |
| FAD-linked oxidoreductase | WP_084705378.1 | Leucobacter chironomi | RWNTSWSNWARTERS | 25 |
| RNA-binding protein 48 | XP_020288140.1 | Pseudomyrmex gracilis | NWNTSWSNTASGSDS | 26 |
| TonB-dependent receptor | WP_083710929.1 | Proteiniphilum saccharofermentans | SINAAWSNQSYGFSR | 27 |
| Peptide ABC transporter | WP_074918487.1 | Terrisporobacter glycolicus | NNNAQWSNKEYDKIV | 28 |
| Type IV secretion protein Rhs | WP_032559173.1 | Bacteroides fragilis | HTCASWCNKSLSDIV | 29 |
| Putative transmembrane rhomboid family protein | CAH09895.1 | Bacteroides fragilis | KDITSWVNKALDAIA | 30 |
| Receptor kinase-like protein Xa21 | XP_020096846.1 | Ananas comosus | GALSSWSNKSLHCCE | 31 |
| Rim ABC transporter | AAC05632.1 | Homo sapiens | EKVANWSIKSLGLTV | 32 |
| Importin beta SMX1 | KTB14942.1 | Candida glabrata | KFIESWSNKSL WLGE | 33 |
| Transporter | WP_049174838.1 | Acinetobacter ursingii | FLLYALSNKSLNDIW | 34 |
| Sugar ABC transporter permease | WP_075333493.1 | Pseudonocardia sp. | PTSVSWSNYEQILVG | 35 |
| ABC transporter substrate binding protein | WP_009846178.1 | Vibrio sp. | DEQKQWRNKSLEQLW | 36 |
| Tetraspanin | NP_001024415.2 | Caenorhabditis elegans | YLGVSWSNKSLLYSY | 37 |
| Nucleotidyltransferase | WP_061886850.1 | Aggregatibacter actinomycetemcomitans | MSKFGLSDKSIEQIH | 38 |
| Phospholipase C, phosphocholine-specific | WP_026813378.1 | Arenibacter certesii | MRYTVESGKSLDDIW | 39 |
| Response regulator of zinc sigma-54-dependent two-component system | WP_087741064. 1 | Proteus mirabilis | FELVCASNKSLEQLA | 40 |
| UNKNOWN | WP_018028689.1 | Porphyromonas somerae | DHDKGLETESLEQIW | 41 |
| Peptide ABC transporter permease | WP_065295736.1 | Aggregatibacter aphrophilus | EPKDFRESATLNQIW | 42 |

**Table 2**

| **Pathogen** | **Ligand** | **ID NO:** |
|---|---|---|
| Virus | HIV gp41 | SEQ ID NO :3 |
| | HIV rev protein | 51 |
| | HCV core protein (a.a 26-124) | SEQ ID NO: 43 |
| | EBV EBNA1 | 45 |
| | Adenovirus core protein V | 46 |
| | Hantaan virus (HTNV) capsid | 47 |
| | HSV Neurovirulence factor ICP34 | 48 |
| | Rubella virus protease p150 | 49 |
| | Rubella virus capsid protein | 50 |
| Bacteria | Staphylococcus aureus protein A | SEQ ID NO : 44 |
| | Internalin InIB Listeria monocytogenes | 52 |
| | Streptococcus pneumoniae hyaluronate lyase | 53 |
| | Exosporium of Bacillus cereus | 54 |
| Parasite | Plasmodium falciparum | 55 |
| Artifical ligand | LvP-1 | 56 |
| Serum components | C1q | 57 |
| | Kininogen | 58 |
| | Vitronectin | 59 |
| | Hyaluronan | 60 |
| | Tissue factor pathway inhibitor-2 (TFPI-2) | 61 |
| Co-receptor | DC-SIGN (CD209) | 62 |
| Mitochondrial protein | Mitochondrial antiviral-signaling protein (MAVS) | 63 |
| Cytosol/nuclear | Nucleus-related like TFII B | 64 |
| | Laminin B receptor p58 | 65 |
| | splicing factor-2 (ASF/SF2) | 66 |
| | Cytokeratin 1 | 67 |
| | CDKN2A isoform smARF | 68 |
| | PPIF | 69 |
| | U2AF1L4 | 70 |
| | Nop52 | 71 |

**Table 3**

| **PROTEIN** | **SEQUENCES** | **SEQ ID NO :** | **PATHOLOGY** |
|---|---|---|---|
| TonB-dependent receptor | SFLKSWFNNSLVDIG | 7 | Pancreatic cancer, Chronic periodontal disease Rheumatoid polyarthritis, Alzheimer's Disease |
| UNKNOWN | SGEGGWSNGSLVDIM | 8 | |
| MC73_05565 | PWNAIWSAKNTTVDS | 14 | Urinary tract infections, Osteomyelitis in a HIV-patient |
| LVIVD repeat protein | PWNASWSYVLDSAWS | 18 | Leptospirosis |
| Peptide ABC transporter | NNNAQWSNKEYDKIV | 28 | Wounds infection |
| Type IV secretion protein RhS | HTCASWCNKSLSDIV | 29 | Peritoneal infections, bacteremia, subcutaneous abscesses or burns |
| putative transmembrane rhomboid family protein | KDITSWVNKALDAIA | 30 | |
| Importin beta SMX1 | KFIESWSNKSLWLGE | 33 | Cutaneous candidiasis in HIV/AIDS, cancer and organ transplantation |
| Nucleotidyltransferase | MSKFGLSDKSIEQIH | 38 | Agressive Periodontitis, Bacterial vaginosis, Endocarditis, actinomycosis, Rheumatoid arthritis |
| UNKNOWN | DHDKGLETESLEQIW | 41 | Chronic skin, soft tissue and bone infections |
| Peptide ABC transporter permease | EPKDFRESATLNQIW | 42 | Endocarditis, brain abscesses, vertebral osteomyelitis and bacteremia |

### REFERENCES

Ghebrehiwet B, Jesty J, Vinayagasundaram R, Vinayagasundaram U, Ji Y, Valentino A, Tumma N, Hosszu KH, Peerschke EI. Targeting gClqR domains for therapy against infection and inflammation. Adv Exp Med Biol. 2013;735:97-110.
Rossio JL, Esser MT, Suryanarayana K, Schneider DK, Bess JW Jr, Vasquez GM, Wiltrout TA, Chertova E, Grimes MK, Sattentau Q, Arthur LO, Henderson LE, Lifson JD. Inactivation of human immunodeficiency virus type 1 infectivity with preservation of conformational and functional integrity of virion surface proteins. J Virol. 1998 Oct; 72(10):7992-8001.
Platt EJ, Wehrly K, Kuhmann SE, Chesebro B, Kabat D. Effects of CCR5 and CD4 cell surface concentrations on infections by macrophagetropic isolates of human immunodeficiency virus type 1. J Virol. 1998 Apr;72(4):2855-64.
Vieillard V, Strominger JL, Debré P. NK cytotoxicity against CD4+ T cells during HIV-1 infection: A gp41 peptide induces expression of an NKp44 ligand. PNAS 2005 Aug 2;102(31):10981-6.
Fausther-Bovendo H, Vieillard V, Sagan S, Bismuth G, Debré P. HIV gp41 engages gClqR on CD4+ T cells to induce the expression of an NK ligand through the PIP3/H2O2 pathway. PLoS Pathog. 2010 Jul 1;6:e1000975.
Kittlesen DJ, Chianese-Bullock KA, Yao ZQ, Braciale TJ, Hahn YS. Interaction between complement receptor gClqR and hepatitis C virus core protein inhibits T-lymphocyte proliferation. J Clin Invest. 2000 Nov;106(10):1239-49.
Large MK, Kittlesen DJ, Hahn YS. Suppression of host immune response by the core protein of hepatitis C virus: possible implications for hepatitis C virus persistence. J Immunol. 1999 Jan 15;162(2):931-8.

## Claims

1. A PLA2-GIB cofactor, or a modulator of a PLA2-GIB cofactor, for use for treating a mammalian subject.

2. A PLA2-GIB cofactor, or a modulator of a PLA2-GIB co-factor, for use for modulating an immune response in a mammalian subject.

3. The cofactor or modulator for use according to claim 1 or 2, wherein the PLA2-GIB cofactor is a ligand of gC1qR.

4. The cofactor or modulator for use according to claim 1 or 2, wherein the PLA2-GIB cofactor is a protein selected from the proteins of Table 1 or 2, or a gClqR-binding element of such a protein.

5. The cofactor or modulator for use according to anyone of claims 1 to 4, wherein the PLA2-GIB cofactor is a component of a pathogen or a nutrient, preferably a protein or peptide from a pathogen.

6. The cofactor or modulator for use according to claim 5, wherein the PLA2-GIB cofactor is a viral or bacterial or fungal or parasite protein or peptide.

7. The cofactor or modulator for use according to claim 6, wherein the PLA2-GIB cofactor is HCV core protein or Staphylococcus protein A, or a fragment or mimotope thereof.

8. A cofactor or modulator for use according to anyone of claims 1 to 7, wherein the modulator of PLA2-GIB cofactor is an inhibitor of the PLA2-GIB cofactor.

9. A cofactor or modulator for use according to claim 8, wherein the inhibitor inhibits binding of the cofactor to gClqR.

10. A cofactor or modulator for use according to claim 8, wherein the inhibitor inhibits expression of the cofactor.

11. A cofactor or modulator for use according to claim 9, wherein the inhibitor is a compound which binds to gClqR or to the cofactor, and inhibits a function of gClqR.

12. The cofactor or modulator for use according to claim 11, wherein the inhibitor inhibits gC1qR-mediated exocytosis.

13. The cofactor or modulator for use according to any one of claims 8 to 12, wherein the inhibitor is an antibody or a variant or fragment of an antibody.

14. The cofactor or modulator for use according to claim 13, wherein the inhibitor is an antibody, or a variant or fragment thereof, which binds gC1qR or a protein selected from Table 1 or 2, and preferably inhibits binding of said protein to gClqR.

15. The cofactor or modulator for use according to any one of claims 8 to 12, wherein the inhibitor is a peptide or lipopeptide.

16. The cofactor or modulator for use according to claim 15, wherein the inhibitor is a peptide which binds gClqR and inhibits binding to gClqR of a protein selected from Table 1 or 2.

17. The cofactor or modulator for use according to any one of claims 8 to 12, wherein the inhibitor is a nucleic acid.

18. The cofactor or modulator for use according to any one of claims 8 to 12, wherein the inhibitor is a carbohydrate.

19. The cofactor or modulator for use according to any one of claims 1 to 7, wherein the modulator of PLA2-GIB cofactor is an immunogen of the PLA2-GIB cofactor, which can induce antibodies to the cofactor.

20. The cofactor or modulator for use according to any one of claims 8 to 19, wherein the subject has a disease caused by a pathogenic agent, preferably an infectious pathogen.

21. The cofactor or modulator for use according to claim 20, wherein the infectious pathogen is a virus or a bacterium or a fungus or a parasite.

22. The cofactor or modulator for use according to any one of claims 8 to 19, wherein the subject has a disease caused by or associated with or inducing an immunodepression.

23. The cofactor or modulator for use according to anyone of claims 8 to 22, wherein the cofactor modulator is administered in combination with another drug or treatment.

24. A cofactor or modulator for use according to claim 1, for treating a disease caused by a pathogenic bacterium in a subject, the method comprising administering to the subject an inhibitor of a PLA2-GIB cofactor.

25. A cofactor or modulator for use according to claim 1, for treating a disease caused by a virus in a subject, the method comprising administering to the subject an inhibitor of a PLA2-GIB cofactor.

26. A cofactor or modulator for use according to claim 25, for treating a disease caused by a hepatitis virus or HIV in a subject, the method comprising administering to the subject an inhibitor of PLA2-GIB cofactor.

27. A cofactor or modulator for use according to claim 1 or 2, wherein the modulator of the PLA2-GIB cofactor is an activator or agonist or mimotope of the PLA2-GIB cofactor.

28. The cofactor or modulator for use according to any one of claims 1 to 27, wherein the subject has a condition requiring immunosuppressive or immunostimulating therapy.

29. A PLA2-GIB cofactor, or an agonist or mimotope thereof, for use to induce immunosuppression in a subject in need thereof by increasing the effect of PLA2-GIB on T cells.

30. The cofactor or modulator for use according to anyone of the preceding claims, wherein the subject is a mammal, preferably a human.

31. A combination therapy or therapeutic regimen for treating a disorder caused by a pathogen, comprising a combination of at least two active agents selected from (i) a drug active against the pathogen, (ii) a modulator of a PLA2-GIB cofactor, and (iii) a modulator of PLA2-GIB, said drug and modulator being for combined, separate or sequential administration.


SEQUENCE LISTING

<110>  DIACCURATE

<120>  THERAPEUTIC METHODS

<130>  B2696

<160>  44

<170>  PatentIn version 3.5

<210>  1
<211>  148
<212>  PRT
<213>  Artificial

<220>
<223>  PLA2-GIB

<400>  1

Met Lys Leu Leu Val Leu Ala Val Leu Leu Thr Val Ala Ala Ala Asp
1               5                   10                  15

Ser Gly Ile Ser Pro Arg Ala Val Trp Gln Phe Arg Lys Met Ile Lys
            20                  25                  30

Cys Val Ile Pro Gly Ser Asp Pro Phe Leu Glu Tyr Asn Asn Tyr Gly
        35                  40                  45

Cys Tyr Cys Gly Leu Gly Gly Ser Gly Thr Pro Val Asp Glu Leu Asp
    50                  55                  60

Lys Cys Cys Gln Thr His Asp Asn Cys Tyr Asp Gln Ala Lys Lys Leu
65                  70                  75                  80

Asp Ser Cys Lys Phe Leu Leu Asp Asn Pro Tyr Thr His Thr Tyr Ser
                85                  90                  95

Tyr Ser Cys Ser Gly Ser Ala Ile Thr Cys Ser Ser Lys Asn Lys Glu
            100                 105                 110

Cys Glu Ala Phe Ile Cys Asn Cys Asp Arg Asn Ala Ala Ile Cys Phe
        115                 120                 125

Ser Lys Ala Pro Tyr Asn Lys Ala His Lys Asn Leu Asp Thr Lys Lys
    130                 135                 140

Tyr Cys Gln Ser
145

<210>  2

<211>  282
<212>  PRT
<213>  Artificial

<220>
<223>  gC1qR

<400>  2

Met Leu Pro Leu Leu Arg Cys Val Pro Arg Val Leu Gly Ser Ser Val
1               5                   10                  15

Ala Gly Leu Arg Ala Ala Ala Pro Ala Ser Pro Phe Arg Gln Leu Leu
            20                  25                  30

Gln Pro Ala Pro Arg Leu Cys Thr Arg Pro Phe Gly Leu Leu Ser Val
        35                  40                  45

Arg Ala Gly Ser Glu Arg Arg Pro Gly Leu Leu Arg Pro Arg Gly Pro
    50                  55                  60

Cys Ala Cys Gly Cys Gly Cys Gly Ser Leu His Thr Asp Gly Asp Lys
65                  70                  75                  80

Ala Phe Val Asp Phe Leu Ser Asp Glu Ile Lys Glu Glu Arg Lys Ile
                85                  90                  95

Gln Lys His Lys Thr Leu Pro Lys Met Ser Gly Gly Trp Glu Leu Glu
            100                 105                 110

Leu Asn Gly Thr Glu Ala Lys Leu Val Arg Lys Val Ala Gly Glu Lys
        115                 120                 125

Ile Thr Val Thr Phe Asn Ile Asn Asn Ser Ile Pro Pro Thr Phe Asp
    130                 135                 140

Gly Glu Glu Glu Pro Ser Gln Gly Gln Lys Val Glu Glu Gln Glu Pro
145                 150                 155                 160

Glu Leu Thr Ser Thr Pro Asn Phe Val Val Glu Val Ile Lys Asn Asp
                165                 170                 175

Asp Gly Lys Lys Ala Leu Val Leu Asp Cys His Tyr Pro Glu Asp Glu
            180                 185                 190

Val Gly Gln Glu Asp Glu Ala Glu Ser Asp Ile Phe Ser Ile Arg Glu
        195                 200                 205

Val Ser Phe Gln Ser Thr Gly Glu Ser Glu Trp Lys Asp Thr Asn Tyr
    210                 215                 220

Thr Leu Asn Thr Asp Ser Leu Asp Trp Ala Leu Tyr Asp His Leu Met
225                 230                 235                 240

Asp Phe Leu Ala Asp Arg Gly Val Asp Asn Thr Phe Ala Asp Glu Leu
                245                 250                 255

Val Glu Leu Ser Thr Ala Leu Glu His Gln Glu Tyr Ile Thr Phe Leu
            260                 265                 270

Glu Asp Leu Lys Ser Phe Val Lys Ser Gln
        275                 280

<210>  3
<211>  344
<212>  PRT
<213>  Artificial

<220>
<223>  PLA2-GIB cofactor

<400>  3

Ala Ala Ile Gly Ala Leu Phe Leu Gly Phe Leu Gly Ala Ala Gly Ser
1               5                   10                  15

Thr Met Gly Ala Ala Ser Val Thr Leu Thr Val Gln Ala Arg Leu Leu
            20                  25                  30

Leu Ser Gly Ile Val Gln Gln Gln Asn Asn Leu Leu Arg Ala Ile Glu
        35                  40                  45

Ser Gln Gln His Met Leu Arg Leu Thr Val Trp Gly Ile Lys Gln Leu
    50                  55                  60

Gln Ala Arg Val Leu Ala Val Glu Arg Tyr Leu Lys Asp Gln Gln Leu
65                  70                  75                  80

Leu Gly Phe Trp Gly Cys Ser Gly Lys Leu Ile Cys Thr Thr Thr Val
                85                  90                  95

Pro Trp Asn Ala Ser Trp Ser Asn Lys Ser Leu Asp Asp Ile Trp Asn
            100                 105                 110

Asn Met Thr Trp Met Gln Trp Glu Arg Glu Ile Asp Asn Tyr Thr Ser
        115                 120                 125

Leu Ile Tyr Ser Leu Leu Glu Lys Ser Gln Thr Gln Gln Glu Lys Asn
    130                 135                 140

Glu Gln Glu Leu Leu Glu Leu Asp Lys Trp Ala Ser Leu Trp Asn Trp
145                 150                 155                 160

Phe Asp Ile Thr Asn Trp Leu Trp Tyr Ile Lys Ile Phe Ile Met Ile
                165                 170                 175

Val Gly Gly Leu Val Gly Leu Arg Ile Val Phe Ala Val Leu Ser Ile
            180                 185                 190

Val Asn Arg Val Arg Gln Gly Tyr Ser Pro Leu Ser Leu Gln Thr Arg
        195                 200                 205

Pro Pro Val Pro Arg Gly Pro Asp Arg Pro Glu Gly Ile Glu Glu Glu
    210                 215                 220

Gly Gly Glu Arg Asp Arg Asp Thr Ser Gly Arg Leu Val His Gly Phe
225                 230                 235                 240

Leu Ala Ile Ile Trp Val Asp Leu Arg Ser Leu Phe Leu Leu Ser Tyr
                245                 250                 255

His His Leu Arg Asp Leu Leu Leu Ile Ala Ala Arg Ile Val Glu Leu
            260                 265                 270

Leu Gly Arg Arg Gly Trp Glu Val Leu Lys Tyr Trp Trp Asn Leu Leu
        275                 280                 285

Gln Tyr Trp Ser Gln Glu Leu Lys Ser Ser Ala Val Ser Leu Leu Asn
    290                 295                 300

Ala Ala Ala Ile Ala Val Ala Glu Gly Thr Asp Arg Val Ile Glu Val
305                 310                 315                 320

Leu Gln Arg Ala Gly Arg Ala Ile Leu His Ile Pro Thr Arg Ile Arg
                325                 330                 335

Gln Gly Leu Glu Arg Ala Leu Leu
            340

<210>  4
<211>  15
<212>  PRT
<213>  Artificial

<220>
<223>  PLA2-GIB cofactor

<400>  4

Ala Trp Asn Ala Ile Trp Ile Asn Arg Lys Tyr Glu Gln Ile Asp
1               5                   10                  15

<210>  5
<211>  15
<212>  PRT
<213>  Artificial

<220>
<223>  PLA2-GIB cofactor

<400>  5

Gly Ile Ala Glu Ser Trp Pro Asn Ser Leu Asp Asp Ser Cys Ala
1               5                   10                  15

<210>  6
<211>  15
<212>  PRT
<213>  Artificial

<220>
<223>  PLA2-GIB cofactor

<400>  6

Gly Ile Ala Glu Ser Trp Pro Asn Ser Leu Asp Asp Ser Cys Ala
1               5                   10                  15

<210>  7
<211>  15
<212>  PRT
<213>  Artificial

<220>
<223>  PLA2-GIB cofactor

<400>  7

Ser Phe Leu Lys Ser Trp Phe Asn Asn Ser Leu Val Asp Ile Gly
1               5                   10                  15

<210>  8
<211>  15
<212>  PRT
<213>  Artificial

<220>
<223>  PLA2-GIB cofactor

<400>  8

Ser Gly Glu Gly Gly Trp Ser Asn Gly Ser Leu Val Asp Ile Met
1               5                   10                  15

<210>  9
<211>  15
<212>  PRT

<213>  Artificial

<220>
<223>  PLA2-GIB cofactor

<400>  9

Tyr Glu Leu Asp Ala Ala Ser Asn Asn Ser Val Asp Asp Ile Arg
1               5                   10                  15


<210>  10
<211>  15
<212>  PRT
<213>  Artificial

<220>
<223>  PLA2-GIB cofactor

<400>  10

Ala Ala Leu Gly Lys Thr Leu Val Lys Ser Leu Asp Asp Ile Pro
1               5                   10                  15


<210>  11
<211>  15
<212>  PRT
<213>  Artificial

<220>
<223>  PLA2-GIB cofactor

<400>  11

Pro Trp Asn Ala Ser Trp Ser Asp Lys Phe Tyr Glu Asn Ser Leu
1               5                   10                  15


<210>  12
<211>  15
<212>  PRT
<213>  Artificial

<220>
<223>  PLA2-GIB cofactor

<400>  12

Ala Ile His Ala Ser Trp Ser Asn Thr Ser Tyr Glu Val Ile Asp
1               5                   10                  15


<210>  13
<211>  15
<212>  PRT
<213>  Artificial

<220>
<223>  PLA2-GIB cofactor

<400>  13

Pro Trp Asn Ala Gly Trp Ser Asn Ala Arg Phe Asp Glu Leu Cys
1               5                   10                  15


<210>  14
<211>  15
<212>  PRT
<213>  Artificial

<220>
<223>  PLA2-GIB cofactor

<400>  14

Pro Trp Asn Ala Ile Trp Ser Ala Lys Asn Thr Thr Val Asp Ser
1               5                   10                  15


<210>  15
<211>  15
<212>  PRT
<213>  Artificial

<220>
<223>  PLA2-GIB cofactor

<400>  15

Pro Trp Asn Ala Ser Trp Ser Ala Ala Gly Val Gly Ala His Ala
1               5                   10                  15


<210>  16
<211>  15
<212>  PRT
<213>  Artificial

<220>
<223>  PLA2-GIB cofactor

<400>  16

Trp Phe Asn Ala Ser Trp Lys Asp Lys Ser Tyr Ser Thr Val Trp
1               5                   10                  15


<210>  17
<211>  15
<212>  PRT
<213>  Artificial

<220>
<223>  PLA2-GIB cofactor

<400>  17

Pro Trp Asn Ala Ser Trp Ser Val Arg His Ile Ser Glu Leu Glu
1               5                   10                  15


<210>  18
<211>  15
<212>  PRT

<213>  Artificial

<220>
<223>  PLA2-GIB cofactor

<400>  18

Pro Trp Asn Ala Ser Trp Ser Tyr Val Leu Asp Ser Ala Trp Ser
1               5                   10                  15


<210>  19
<211>  15
<212>  PRT
<213>  Artificial

<220>
<223>  PLA2-GIB cofactor

<400>  19

Pro Trp Asn Gly Ser Trp Ser Asn Asp Ala Trp Gly Pro Gly Thr
1               5                   10                  15


<210>  20
<211>  15
<212>  PRT
<213>  Artificial

<220>
<223>  PLA2-GIB cofactor

<400>  20

Pro Trp Asn Ala Gly Trp Ser Asn Ala Arg Phe Asp Glu Leu Cys
1               5                   10                  15


<210>  21
<211>  15
<212>  PRT
<213>  Artificial

<220>
<223>  PLA2-GIB cofactor

<400>  21

Pro Trp Asn Ala Gly Trp Ser Leu Lys Ser Ser Gly Lys Ser Ala
1               5                   10                  15


<210>  22
<211>  15
<212>  PRT
<213>  Artificial

<220>
<223>  PLA2-GIB cofactor

<400>  22

Pro Trp Asn Ala Trp Trp Ser Asn Arg Ser Met Ile Ala Asp Val
1               5                   10                  15


<210>  23
<211>  15
<212>  PRT
<213>  Artificial

<220>
<223>  PLA2-GIB cofactor

<400>  23

Ala Trp Asn Glu Ser Trp Ser Asn Lys Ser Phe His Asn Gly Ala
1               5                   10                  15


<210>  24
<211>  15
<212>  PRT
<213>  Artificial

<220>
<223>  PLA2-GIB cofactor

<400>  24

Glu Phe Asn Ala Asn Trp Ser Asn Lys Phe Tyr Leu Tyr Asn Gln
1               5                   10                  15


<210>  25
<211>  15
<212>  PRT
<213>  Artificial

<220>
<223>  PLA2-GIB cofactor

<400>  25

Arg Trp Asn Thr Ser Trp Ser Asn Trp Ala Arg Thr Glu Arg Ser
1               5                   10                  15


<210>  26
<211>  15
<212>  PRT
<213>  Artificial

<220>
<223>  PLA2-GIB cofactor

<400>  26

Asn Trp Asn Thr Ser Trp Ser Asn Thr Ala Ser Gly Ser Asp Ser
1               5                   10                  15


<210>  27
<211>  15
<212>  PRT

<213>  Artificial

<220>
<223>  PLA2-GIB cofactor

<400>  27

Ser Ile Asn Ala Ala Trp Ser Asn Gln Ser Tyr Gly Phe Ser Arg
1               5                   10                  15


<210>  28
<211>  15
<212>  PRT
<213>  Artificial

<220>
<223>  PLA2-GIB cofactor

<400>  28

Asn Asn Asn Ala Gln Trp Ser Asn Lys Glu Tyr Asp Lys Ile Val
1               5                   10                  15


<210>  29
<211>  15
<212>  PRT
<213>  Artificial

<220>
<223>  PLA2-GIB cofactor

<400>  29

His Thr Cys Ala Ser Trp Cys Asn Lys Ser Leu Ser Asp Ile Val
1               5                   10                  15


<210>  30
<211>  15
<212>  PRT
<213>  Artificial

<220>
<223>  PLA2-GIB cofactor

<400>  30

Lys Asp Ile Thr Ser Trp Val Asn Lys Ala Leu Asp Ala Ile Ala
1               5                   10                  15


<210>  31
<211>  15
<212>  PRT
<213>  Artificial

<220>
<223>  PLA2-GIB cofactor

<400>  31

Gly Ala Leu Ser Ser Trp Ser Asn Lys Ser Leu His Cys Cys Glu
1               5                   10                  15


<210>  32
<211>  15
<212>  PRT
<213>  Artificial

<220>
<223>  PLA2-GIB cofactor

<400>  32

Glu Lys Val Ala Asn Trp Ser Ile Lys Ser Leu Gly Leu Thr Val
1               5                   10                  15


<210>  33
<211>  15
<212>  PRT
<213>  Artificial

<220>
<223>  PLA2-GIB cofactor

<400>  33

Lys Phe Ile Glu Ser Trp Ser Asn Lys Ser Leu Trp Leu Gly Glu
1               5                   10                  15


<210>  34
<211>  15
<212>  PRT
<213>  Artificial

<220>
<223>  PLA2-GIB cofactor

<400>  34

Phe Leu Leu Tyr Ala Leu Ser Asn Lys Ser Leu Asn Asp Ile Trp
1               5                   10                  15


<210>  35
<211>  15
<212>  PRT
<213>  Artificial

<220>
<223>  PLA2-GIB cofactor

<400>  35

Pro Thr Ser Val Ser Trp Ser Asn Tyr Glu Gln Ile Leu Val Gly
1               5                   10                  15


<210>  36
<211>  15
<212>  PRT

<213>  Artificial

<220>
<223>  PLA2-GIB cofactor

<400>  36

Asp Glu Gln Lys Gln Trp Arg Asn Lys Ser Leu Glu Gln Leu Trp
1               5                   10                  15


<210>  37
<211>  15
<212>  PRT
<213>  Artificial

<220>
<223>  PLA2-GIB cofactor

<400>  37

Tyr Leu Gly Val Ser Trp Ser Asn Lys Ser Leu Leu Tyr Ser Tyr
1               5                   10                  15


<210>  38
<211>  15
<212>  PRT
<213>  Artificial

<220>
<223>  PLA2-GIB cofactor

<400>  38

Met Ser Lys Phe Gly Leu Ser Asp Lys Ser Ile Glu Gln Ile His
1               5                   10                  15


<210>  39
<211>  15
<212>  PRT
<213>  Artificial

<220>
<223>  PLA2-GIB cofactor

<400>  39

Met Arg Tyr Thr Val Glu Ser Gly Lys Ser Leu Asp Asp Ile Trp
1               5                   10                  15


<210>  40
<211>  15
<212>  PRT
<213>  Artificial

<220>
<223>  PLA2-GIB cofactor

<400>  40

Phe Glu Leu Val Cys Ala Ser Asn Lys Ser Leu Glu Gln Leu Ala
1               5                   10                  15


<210>  41
<211>  15
<212>  PRT
<213>  Artificial

<220>
<223>  PLA2-GIB cofactor

<400>  41

Asp His Asp Lys Gly Leu Glu Thr Glu Ser Leu Glu Gln Ile Trp
1               5                   10                  15


<210>  42
<211>  15
<212>  PRT
<213>  Artificial

<220>
<223>  PLA2-GIB cofactor

<400>  42

Glu Pro Lys Asp Phe Arg Glu Ser Ala Thr Leu Asn Gln Ile Trp
1               5                   10                  15


<210>  43
<211>  191
<212>  PRT
<213>  Artificial

<220>
<223>  PLA2-GIB cofactor


<220>
<221>  misc_feature
<222>  (180)..(180)
<223>  Xaa can be any naturally occurring amino acid

<400>  43

Met Ser Thr Asn Pro Lys Pro Gln Arg Lys Thr Lys Arg Asn Thr Ile
1               5                   10                  15


Arg Arg Pro Gln Asp Val Lys Phe Pro Gly Gly Gly Gln Ile Val Gly
            20                  25                  30


Gly Val Tyr Leu Leu Pro Arg Arg Gly Pro Arg Leu Gly Val Arg Ala
        35                  40                  45


Thr Arg Lys Thr Ser Glu Arg Ser Gln Pro Arg Gly Arg Arg Gln Pro
    50                  55                  60

Ile Pro Lys Ala Arg Arg Pro Glu Gly Arg Thr Trp Ala Gln Pro Gly
65                  70                  75                  80

Tyr Pro Trp Pro Leu Tyr Gly Asn Glu Gly Met Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Val Pro Leu Val Gly Ala Pro Leu
    130                 135                 140

Gly Gly Ala Ala Arg Ala Leu Ala His Gly Val Arg Ala Leu Glu Asp
145                 150                 155                 160

Gly Val Asn Tyr Ala Thr Gly Asn Leu Pro Gly Cys Ser Phe Ser Ile
                165                 170                 175

Ser Leu Trp Xaa Leu Leu Ser Cys Leu Thr Ile Pro Ala Ser Ala
            180                 185                 190

<210>  44
<211>  516
<212>  PRT
<213>  Artificial

<220>
<223>  PLA2-GIB cofactor

<400>  44

Met Lys Lys Lys Asn Ile Tyr Ser Ile Arg Lys Leu Gly Val Gly Ile
1               5                   10                  15

Ala Ser Val Thr Leu Gly Thr Leu Leu Ile Ser Gly Gly Val Thr Pro
            20                  25                  30

Ala Ala Asn Ala Ala Gln His Asp Glu Ala Gln Gln Asn Ala Phe Tyr
        35                  40                  45

Gln Val Leu Asn Met Pro Asn Leu Asn Ala Asp Gln Arg Asn Gly Phe
    50                  55                  60

Ile Gln Ser Leu Lys Asp Asp Pro Ser Gln Ser Ala Asn Val Leu Gly
65                  70                  75                  80

Glu Ala Gln Lys Leu Asn Asp Ser Gln Ala Pro Lys Ala Asp Ala Gln
                85                  90                  95

Gln Asn Asn Phe Asn Lys Asp Gln Gln Ser Ala Phe Tyr Glu Ile Leu
            100                 105                 110

Asn Met Pro Asn Leu Asn Glu Ala Gln Arg Asn Gly Phe Ile Gln Ser
        115                 120                 125

Leu Lys Asp Asp Pro Ser Gln Ser Thr Asn Val Leu Gly Glu Ala Lys
    130                 135                 140

Lys Leu Asn Glu Ser Gln Ala Pro Lys Ala Asp Asn Asn Phe Asn Lys
145                 150                 155                 160

Glu Gln Gln Asn Ala Phe Tyr Glu Ile Leu Asn Met Pro Asn Leu Asn
                165                 170                 175

Glu Glu Gln Arg Asn Gly Phe Ile Gln Ser Leu Lys Asp Asp Pro Ser
            180                 185                 190

Gln Ser Ala Asn Leu Leu Ser Glu Ala Lys Lys Leu Asn Glu Ser Gln
        195                 200                 205

Ala Pro Lys Ala Asp Asn Lys Phe Asn Lys Glu Gln Gln Asn Ala Phe
    210                 215                 220

Tyr Glu Ile Leu His Leu Pro Asn Leu Asn Glu Glu Gln Arg Asn Gly
225                 230                 235                 240

Phe Ile Gln Ser Leu Lys Asp Asp Pro Ser Gln Ser Ala Asn Leu Leu
                245                 250                 255

Ala Glu Ala Lys Lys Leu Asn Asp Ala Gln Ala Pro Lys Ala Asp Asn
            260                 265                 270

Lys Phe Asn Lys Glu Gln Gln Asn Ala Phe Tyr Glu Ile Leu His Leu
        275                 280                 285

Pro Asn Leu Thr Glu Glu Gln Arg Asn Gly Phe Ile Gln Ser Leu Lys
    290                 295                 300

Asp Asp Pro Ser Val Ser Lys Glu Ile Leu Ala Glu Ala Lys Lys Leu
305                 310                 315                 320

Asn Asp Ala Gln Ala Pro Lys Glu Glu Asp Asn Asn Lys Pro Gly Lys
                325                 330                 335

Glu Asp Asn Asn Lys Pro Gly Lys Glu Asp Asn Asn Lys Pro Gly Lys
            340                 345                 350

Glu Asp Asn Asn Lys Pro Gly Lys Glu Asp Asn Asn Lys Pro Gly Lys
        355                 360                 365

Glu Asp Gly Asn Lys Pro Gly Lys Glu Asp Asn Lys Lys Pro Gly Lys
    370                 375                 380

Glu Asp Gly Asn Lys Pro Gly Lys Glu Asp Asn Lys Lys Pro Gly Lys
385                 390                 395                 400

Glu Asp Gly Asn Lys Pro Gly Lys Glu Asp Gly Asn Lys Pro Gly Lys
                405                 410                 415

Glu Asp Gly Asn Gly Val His Val Val Lys Pro Gly Asp Thr Val Asn
            420                 425                 430

Asp Ile Ala Lys Ala Asn Gly Thr Thr Ala Asp Lys Ile Ala Ala Asp
        435                 440                 445

Asn Lys Leu Ala Asp Lys Asn Met Ile Lys Pro Gly Gln Glu Leu Val
    450                 455                 460

Val Asp Lys Lys Gln Pro Ala Asn His Ala Asp Ala Asn Lys Ala Gln
465                 470                 475                 480

Ala Leu Pro Glu Thr Gly Glu Glu Asn Pro Phe Ile Gly Thr Thr Val
                485                 490                 495

Phe Gly Gly Leu Ser Leu Ala Leu Gly Ala Ala Leu Leu Ala Gly Arg
            500                 505                 510

Arg Arg Glu Leu
        515